# FASCICULE DE BREVET EUROPEEN

(11) **EP 0 261 016 B1**
(45) Date de publication et mention de la délivrance du brevet: **10.03.1993**
(21) Numéro de dépôt: 87401984.7
(22) Date de dépôt: 04.09.1987
(51) Int. Cl.: C12N 9/20, A61K 37/54

(54) **Lipases et extraits lipasiques, leur procédé de préparation et leur application en thérapeutique**
Lipase und lipasische Extrakte, Verfahren zur Herstellung und therapeutische Anwendung
Lipase and lipasic extracts, process of preparation and therapeutic use

(30) Priorité: 17.09.1986 FR 8612996
(43) Date de publication de la demande: 23.03.1988
(73) Titulaire: JOUVEINAL S.A., F-75755 Paris Cedex 15 (FR)
(72) Inventeur: Moreau, Hervé, F-13008 Marseille (FR); Verger, Robert, F-13009 Marseille (FR); Lecat, Daniel, F-75013 Paris (FR); Junien, Jean-Louis, F-92310 Sevres (FR)
(74) Mandataire: Bourgognon, Jean-Marie

(56) Documents cités:
- WO-A-86/01532
- FR-M- 6 622
- GB-A- 1 139 991
- GB-A- 2 142 337
- US-A- 3 256 150
- CHEMICAL ABSTRACTS, vol. 97, no. 15, 11 octobre 1982, page 477, résumé no. 124832v, Columbus, Ohio, US; J.P. PERRET: "Gastric lipolysis in the young rabbit. Origin and physiological importance of lipase", & J. PHYSIOL. (PARIS) 1982, 78(2), 221-30

## Description

La présente invention concerne des enzymes d'activité lipasique et les extraits qui les contiennent, produits stables et actifs en milieux acides ou voisins de la neutralité, leur procédé de préparation ainsi que leur application en thérapeutique sous forme de médicaments.

Les lipases, enzymes apparentées aux hydrolases, se trouvent dans des organismes aussi divers que les bactéries, les champignons, les plantes et les animaux. Leur importance dans les fonctions de la matière vivante a été reconnue vers 1900 lors de l'étude du phénomène de la digestion. Leur rôle s'est avéré essentiel pour l'assimilation des matières grasses qui représentent une source énergétique considérable dans l'alimentation humaine et animale. Ultérieurement, leurs propriétés ont été utilisées pour des applications thérapeutiques.

Chez l'homme et les mammifères en général, de nombreuses études ont été effectuées. Elles ont montré le rôle catalytique essentiel de ces enzymes lors des phénomènes digestifs où elles interviennent plus particulièrement en hydrolysant les graisses émulsifiées ou non. Ces graisses sont pour une grande partie composées d'esters formés entre des acides gras de poids moléculaires variés et des alcools mono ou polyfonctionnels comme le glycérol dans le cas des triglycérides. La carence ou la déficience de ces systèmes enzymatiques a été fréquemment constatée dans les cas de malabsorption, dont les conséquences, parfois graves, peuvent conduire à des pertes de poids considérables et à des états anorexiques prononcés. Chez l'homme, ces états, qui peuvent atteindre le jeune enfant aussi bien que l'adulte, ont justifié l'utilisation thérapeutique de système enzymatiques exogènes afin de compenser les déficiences naturelles en lipases chez ces malades. Les compositions utilisées ont fait appel à des organes d'animaux ou à leurs extraits dont la sécrétion en lipases est reconnue et qui agissent normalement dans l'organisme pour l'assimilation des graisses. Ces organes sont situés à divers niveaux du tractus bucco-gastro-intestinal, les plus connus sont ceux de la cavité orale et le pancréas.

Les sécrétions de la cavité orale de mammifères contiennent ainsi des enzymes capables d'hydrolyser les esters d'acides gras. Ce sont les estérases prégastriques et les lipases "orales" qui ne diffèrent entre elles que par leur aptitude à être actives sur des substrats hydrosolubles ou non. Ces enzymes et leurs propriétés ont fait l'objet de nombreux travaux résumés par J.H.Nelson et col. (Journal of Dairy Science, 1976, 60 (3) : 327-362).

Leur présence et leur utilité ont été montrées en particulier chez les jeunes animaux encore non sevrés. En conséquence, la commercialisation d'estérases prégastriques obtenues à partir de veaux, de chevreaux et d'agneaux a pris de l'essor. Ces produits, d'abord sous forme d'extraits liquides puis de poudres concentrées, sont utilisés couramment en médicine vétérinaire dans le traitement de diarrhées du veau et aussi en thérapeutique humaine comme dans le brevet des Etats-Unis d'Amérique n^{o} 3.256.150, dans lequel on décrit une méthode de traitement du syndrome de malabsorption au moyen d'une composition sous forme de poudre, administrée par voie orale, composition comprenant en partie des tissus comestibles prélevés dans la cavité orale d'animaux non sevrés, plus particulièrement la langue et les tissus voisins prélevés chez le veau, le chevreau ou l'agneau. Cette dernière utilisation ne semble pas avoir eu beaucoup de succès vraisemblablement à cause des difficultés de collecte de quantités importantes d'organes prélevés après sacrifice de jeunes animaux destinés à l'alimentation et n'ayant pas encore atteint un développement économiquement rentable.

Pour se libérer de ces problèmes pratiques et économiques, on propose au brevet anglais n^{o} 2.142.337 une lipase linguale de rat ou d'homme pour traiter les déficiences en lipase. Ces enzymes sont préparées par génie génétique.

Au niveau duodénal, les lipases du suc pancréatique ont également une activité très importante pour l'assimilation des matières grasses partiellement dégradées on non. C'est ainsi qu'une déficience de sécrétion enzymatique du pancréas peut provoquer des phénomènes de malabsorption qui, pour les matières grasses se traduisent parfois par des anomalies de la digestion et de l'assimilation de ces matières. Chez l'homme, des médicaments de substitution pancréatique administrés par voie orale ont été commercialisés sous forme de compositions à base de pancréas d'animaux dont l'alimentation est comparable à celle de l'homme comme le porc ou d'alimentation de type herbivore comme le boeuf. Toutefois, l'étude des propriétés de ces enzymes, notamment par Gunter Cordes en 1973, a montré la précarité de ces traitements ; les systèmes enzymatiques de ces organes sont rapidement détruits et inactivés en milieu acide à 37°C et, par là-même, incapables d'assurer leur activité après un transit gastrique. Des solutions ont été proposées afin de préserver l'activité de ces médicaments. Au brevet anglais n^{o} 1.139.991, on décrit une composition comprenant des enzymes pancréatiques associées à des sels de propriétés antacides qui ont pour but, en diminuant l'acidité gastrique naturelle de conserver aux enzymes leurs propriétés lors du transit. Une démarche identique est proposée par Regan P.T. et al. (1978), Hubbard V.S. et al. (1980), et Gon P. Bradbear et al. (1981), en utilisant des composés antagonistes des récepteurs H2 comme la cimétidine.

Par ailleurs, on a aussi tenté de préserver l'activité des enzymes en enrobant les médicaments par un pelliculage gastro-résistant, soluble en milieu intestinal et libérant les principes actifs au niveau duodénal ou plus récemment en les incluant dans des microsphères de diamètre voisin de 3 mm.

Ces solutions ne sont que palliatives et présentent des risques d'effets secondaires chez le patient ou une efficacité incertaine.

En effet, les affections pancréatiques nécessitent souvent des traitements de longue durée durant lesquels la diminution répétée de l'acidité gastrique peut provoquer un affaiblissement local des défenses naturelles vis-à-vis des bactéries et de leur prolifération, phénomène décrit par Weber A.M (1982) et dont les conséquences sont dramatiques dans les cas d'intoxication alimentaire.

L'enrobage des produits ne peut, quant à lui, assurer une sécurité totale d'emploi dans certains cas, comme des insuffisances pancréatiques où l'on a constaté au niveau du tractus gastro-intestinal des valeurs de pH irrégulières, parfois anormalement acides, qui sont incompatibles avec la dissolution de la membrane gastro-protectrice au niveau souhaité.

La présence d'activité lipasique dans le contenu gastrique de différentes espèces animales a été également constatée. L'origine de cette activité a été longtemps controversée et attribuée soit à la sécrétion orale transportée par la salive, soit à un reflux duodénal de suc pancréatique. Mais récemment, Fink C.S. et al. (Am. J. Physiol., 1985, 248 : 68-72) ont montré à partir d'une dispersion de glandes gastriques de lapin une activité lipasique dont l'effet est maximum de pH 5,8 à 6,1. Aux Chemical Abstracts vol 97 no 15, 11 octobre 1982, page 477 no 1248320 Columbus Ohio US, on mentionne une activité lipolytique au niveau gastrique chez le lapereau sevré ou non sevré. Cette activité atteint un maximum à un pH de 7. A la demande de brevet WO 86/01532, on décrit la préparation par génie génétique d'une protéine correspondant à une lipase gastrique humaine utile pour traiter les cas de déficience de cette enzyme.

Les divers travaux effectués sur les lipases et les tentatives pour les uti liser montrent clairement l'importance accordée à ces enzymes et, bien que leur présence ait été montrée dans divers organes et sécrétions de mammifères et que leur nécessité pour l'assimilation des matières grasses par l'organisme soit reconnue, on n'a pas proposé jusqu'à présent de lipase ou de composition lipasique de préparation techniquement et économiquement valable qui puisse, par compensation, assurer de façon certaine cette assimilation des matières grasses et notamment des triglycérides à l'ensemble des niveaux du tractus bucco-gasto-intestinal.

La présente invention rompt avec cet état de la technique en ayant pour objet des lipases et des extraits lipasiques dont l'activité est inaltérée après séjour en milieu acide et qui développent de façon efficace leur aptitude à hydrolyser les triglycérides dans des milieux dont le pH varie de 3 à 7, c'est-à-dire dans des milieux d'acidité compatible à celle de leur utilisation dans l'ensemble du tractus bucco-gastro-intestinal.

Les produits de la présente invention présentent donc par rapport à ceux décrits par l'art antérieur un avantage indéniable, notamment en thérapeutique, par leur aptitude à assurer d'une façon fiable durant la digestion l'hydrolyse des triglycérides notamment à chaîne courte au long du tractus compris entre la cavité orale et le duodénum, tout en provenant de matières premières accessibles en grande quantité et à bas prix.

L'invention a donc pour objet un procédé de préparation d'extraits lipasiques ou d'une lipase éventuellement en solutions, caractérisé en ce qu'il consiste :
a) pour obtenir un extrait lipasique :
   - à mettre le fundus d'estomac de lapin ou de cheval adulte en contact avec un milieu aqueux acide ayant un pH de 1,5 à 5, à raison de 1 à 10 parties en volume du milieu acide pour 1 partie en poids de fundus, à une température de 4 à 30°C et pendant une minute à 15 heures pour obtenir des matières solides et une solution aqueuse contenant une partie lipasique,
   - à séparer la solution aqueuse des matières solides pour obtenir une solution aqueuse séparée,
   - à ajouter à la solution aqueuse séparée une quantité suffisante de sel hydrosoluble et à l'y laisser pendant une durée suffisante pour relarguer l'extrait lipasique cherché et pour obtenir une solution surnageante, et
   - à séparer l'extrait lipasique cherché de la solution surnageante et à le recueillir,
b) pour obtenir un extrait lipasique dessalé :
   - à dissoudre l'extrait lipasique dans une phase aqueuse,
   - à filtrer la phase aqueuse sur une membrane ayant un seuil de coupure compris entre 5000 et 10000 daltons pour obtenir une solution de l'extrait lipasique dessalé, et
   - à lyophiliser la solution d'extrait lipasique dessalé en l'extrait lipasique dessalé.
c) pour obtenir un extrait lipasique délipidé
   - à effectuer en outre sur l'une quelconque des parties solides contenant l'extrait lipasique que sont le fundus d'estomac, l'extrait lipasique et l'extrait lipasique dessalé une opération de délipidation.
d) pour obtenir un extrait lipasique enrichi non délipidé :
   - à dissoudre l'extrait lipasique dans un tampon de pH compris entre 2 et 7, pour obtenir une solution tamponnée,
   - à chromatographier la solution tamponnée de pH compris entre 2 et 7 sur un tamis moléculaire dont la limite d'exclusion est supérieure à 1.000.000 daltons et à recueillir la fraction d'élution exclue contenant l'extrait lipasique enrichi non délipidé en solution tamponnée,
   - à filtrer cette fraction d'élution exclue sur une membrane ayant un seuil de coupure de 10.000 daltons pour obtenir une fraction dessalée, et
   - à lyophiliser cette fraction dessalée en l'extrait lipasique enrichi non délipidé cherché.
e) pour obtenir un extrait lipasique enrichi, délipidé :
   e1
      - à dissoudre l'extrait lipasique dans de l'eau pour obtenir une solution aqueuse contenant l'extrait lipasique,
      - à filtrer la solution aqueuse contenant l'extrait lipasique sur une membrane ayant un seuil de coupure compris entre 5000 et 10000 daltons pour obtenir une solution exempte de sel,
      - à adsorber la solution exempte de sel sur un support échangeur d'ions,
      - à désorber du support par un éluant dont on augmente la force ionique progressivement en fonction du temps,
      - à recueillir une fraction d'élution ayant une activité lipasique et contenant l'extrait lipasique enrichi délipidé,
      - à filtrer la fraction délution ayant une activité lipasique sur une membrane ayant un seuil de coupure compris entre 5000 et 10000 daltons pour obtenir une fraction d'élution dessalée, et
      - à lyophiliser cette fraction d'élution dessalée en l'extrait lipasique enrichi délipidé cherché, ou
   e2
      - à chromatographier la solution tamponnée de pH compris entre 2 et 7 obtenue sous d ci-dessus sur un tamis moléculaire,
      - à recueillir une fraction d'élution retenue correspondant à des masses moléculaires comprises entre 30000 et 55000 daltons et contenant l'extrait lipasique enrichi délipidé,
      - à filtrer cette fraction d'élution retenue correspondant à des masses moléculaires comprises entre 30000 et 55000 et contenant l'extrait lipasique enrichi sur une membrane ayant un seuil de coupure compris entre 5000 et 10000 daltons pour obtenir une solution de l'extrait lipasique enrichi délipidé dessalé, et
      - à lyophiliser cette solution de l'extrait lipasique enrichi délipidé dessalé en l'extrait lipasique enrichi délipidé cherché.
f) pour obtenir la lipase :
   f1
      - à partir de la fraction d'élution ayant une activité lipasique et contenant l'extrait lipasique enrichi délipidé obtenu en e1 ci-dessus,
      - à la chromatographier sur un tamis moléculaire,
      - à recueillir une fraction d'élution retenue correspondant à des masses moléculaires comprises entre 45000 et 55000 daltons et contenant la lipase,
      - à filtrer cette fraction d'élution contenant la lipase sur une membrane ayant un seuil de coupure de 5000 à 10000 daltons pour obtenir une solution dessalée contenant la lipase, et
      - à lyophiliser cette solution dessalée contenant la lipase, ou
   f2
      - à partir de la fraction d'élution retenue correspondant à des masses moléculaires comprises entre 30000 et 55000 daltons et contenant l'extrait lipasique enrichi délipidé obtenu en e2 ci-dessus,
      - à l'adsorber sur un support échangeur d'ions,
      - à désorber du support par un éluant dont on augmente la force ionique progressivement en fonction du temps,
      - à recueillir une fraction d'élution ayant une activité lipasique et contenant la lipase,
      - à filtrer la fraction d'élution ayant une activité lipasique et contenant la lipase sur une membrane ayant un seuil de coupure de 5000 à 10000 daltons pour obtenir une fraction d'élution dessalée contenant la lipase, et
      - à lyophiliser cette fraction d'élution dessalée contenant la lipase en la lipase cherchée.

Par "délipidé", on entend, dans le présent mémoire, un produit contenant la lipase suivant l'invenvention, à l'exclusion de lipides.

Essentiellement le procédé comporte une méthode d'extraction A qui permet d'obtenir des extraits d'activité lipasique spécifique comprise de 1 et 100 unités de milligramme de protéine et une méthode de séparation B qui, à partir des extraits précédents, permet d'obtenir des fractions lipasiques enrichies d'activité spécifique supérieure à 100 unités par milligramme de protéine et également, comme terme ultime de la purification, une lipase pure dont l'activité spécifique est voisine de 1 000 unités par mg de protéine.

Les méthodes A et B du procédé ainsi que les étapes de préparation facultative des organes sont décrites dans ce qui suit.

Dans le procédé suivant l'invention, on utilise des lapins adultes, c'est-à-dire des lapins âgés de plus de un mois au moins et, de préférence, âgés d'au moins deux mois. Ces lapins de taille adulte sont destinés à l'alimentation de l'homme, le prélèvement de leur estomac ne diminuant par leur valeur marchande. On n'utilise pas suivant l'invention l'estomac du lapereau sevré ou non sevré, dont on sait qu'il a une activité lipasique à maximum de pH différent de celle de la lipase suivant l'invention et qui poserait des problèmes de coût insurmontables, tant en raison de la petite dimension de l'estomac du lapereau que de l'impossibilité de vendre le reste du lapereau sacrifié.

De même, on n'utilise que des estomacs de chevaux adultes âgés notamment d'au moins un an et, de préférence, d'au moins trois ans.

On a constaté que la lipase cherchée se trouve, chez le lapin, exclusivement dans le fundus de l'estomac qui en est la partie haute suivie en partie basse de l'antre. On a d'ailleurs constaté que la lipase ne se trouve que dans la partie supérieure de la grande courbure du fundus. On peut donc traiter tout l'estomac de lapin ou toute fraction de celui-ci englobant la partie supérieure de la grande courbure.

Il en va de même pour le cheval. La lipase s'y trouve exclusivement dans le fundus.

Comme le fundus est une partie saillante de l'estomac, il est très facile à découper du reste, ce qui facilite les opérations de collecte, de stockage et de transport.

Cette préparation des organes est facultative et peut comprendre différents traitements comme :
- préparation des estomacs,
- sélection et fragmentation des tissus,
- opérations de délipidation.

Le choix de ces traitements étant décidé en fonction de l'état et de la nature des organes disponibles ainsi que de la qualité notamment pureté du produit lipasique désiré. A titre d'exemple, le schéma ci-dessous résume l'ensemble préféré et la succession de ces opérations préliminaires.

ESTOMACS
ELIMINATION
DES SOUILLURES
SELECTION ET FRAGMENTATION
BROYAGE ET DELIPIDATION
METHODE A

La mise en oeuvre de ces opérations préliminaires consiste d'abord à éliminer des estomacs prélevés à l'abattoir, les éventuelles souillures et les parties d'organes adjacents (oesophage, intestin, rate) puis, éventuellement, séparer et éliminer l'antre. Les tissus peuvent être éventuellement congelés et conservés à - 20°C avant la fragmentation.

La fragmentation peut se réaliser manuellement ou avec l'aide d'appareil permettant d'obtenir des morceaux irréguliers dont la dimension maximale est inférieure ou égale à 5 cm.

La mise en oeuvre des étapes de délipidation consiste à homogénéiser les tissus ou les extraits solides obtenus au cours des procédés de l'invention par broyage ou extraction à l'aide d'un solvant hydrophile et de bas point d'ébullition tels que les alcools et les cétones de faible poids moléculaire. L'acétone est, dans ce dernier cas, le solvant le plus utilisés ; il permet à la fois une éventuelle déshydratation et une délipidation partielles du produit traité.

Les opérations d'homogénéisation ainsi d'ailleurs que toutes les opérations effectuées lors du procédé du préparation des produits de l'invention s'effectuent à des températures inférieures à 25°C et, de préférence, inférieures à 10°C afin d'éviter au mieux la dénaturation des principes actifs, ces dénaturations irréversibles pouvant être d'ordre physiocochimique ou chimique selon les conditions de l'extraction et la nature du solvant utilisé.

Généralement, pour homogénéiser par broyage les organes, on utilise de 1 à 10 parties de solvant pour 1 partie en poids de produit à traiter.

Le broyage est effectué à une température inférieure à 25°C dans des appareils efficaces type "warring blender" afin d'obtenir une dispersion de particules de tissus de taille inférieure à 2 mm. Avec le type d'appateil déjà cité, ce résultat est obtenu après broyage pendant 30 secondes à 5 minutes et à une vitesse de 10 000 à 30 000 tpm.

La fraction insoluble est séparée par décantation, filtration ou centrifugation. On utilise le plus souvent la filtration sous un vide industriel d'environ 50 millibars. Dans ce cas, l'insoluble pulvérulent obtenu est un matériau de constitution homogène, délipidé qui peut être :
- soit directement engagé dans la méthode d'extraction A,
- soit, après élimination du solvant résiduel sous un vide de 20 millibars environ et à une température inférieure à 25°C, être engagé dans des opérations de délipidation plus poussées ou éventuellement être utilisé en temps qu'extrait lipasique partiellement délipidé obtenu par le procédé de l'invention.

Ces opérations consistent à amener les produits au degré de délipidation souhaité en les traitant par des solvants aptes à solubiliser les matières grasses comme les hydrocarbures, les éthers, les cétones, les alcools ou encore les halogénures de carbone, qui peuvent être utilisés seuls ou en mélanges. Ces solvants doivent être facilement éliminables et leur point d'ébullition est voisin ou inférieur à 80°C. Ce sont par exemple les pentanes, les hexanes, les éthers de pétrole, l'éther diéthylique, le tétrahydrofuranne, le chloroforme, le chlorure de méthylène, le tétrachlorure de carbone et le dichloroéthane.

Les opérations de délipidation peuvent comprendre pour un même traitement plusieurs extractions successives avec le même solvant ou des solvants différents jusqu'à obtention d'un produit suffisamment délipidé.

La teneur en lipide des extraits lipasiques solides des produits préparés selon l'invention est déterminée par une variante de la méthode précédemment décrite. Pratiquement après dessication d'une prise d'essai du produit à 100-105°C pendant 6 heures ; les lipides sont extraits par un mélange méthanol chloroforme (25 : 75 v/v) durant 3 heures à température ambiante. L'insoluble est filtré, les solvants éliminés par évaporation. Le poids de résidu d'évaporation obtenu rapporté au poids de la prise d'essai de départ permet de déterminer la teneur en lipide, généralement celle-ci est inférieure à 1 % pour les extraits délipidés.

Ce taux de délipidation est déterminé selon les formes prévues pour le produit final, qui peuvent être par exemple comprimée ou pulvérulentes et être réalisées avec des produits de l'invention de qualité organoleptique différente. Le taux de délipidation peut être estimé en déterminant dans un essai préalable la totalité des substances grasses de délipidation à froid la quantité extraite sur une fraction aliquote de la phase liquide.

L'opération en elle-même consiste à mélanger intimement dans un appareil approprié une partie en poids du matériau à traiter avec de 5 à 100 parties du solvant de délipidation choisi, pendant une durée de 15 secondes à 15 minutes et à température inférieure à 25°C. Le produit délipidé est séparé du solvant par filtration ou centrifugation et il peut être retraité de façon analogue autant de fois qu'il est nécessaire pour obtenir un degré de délipidation désiré.

Ainsi, d'une façon plus précise et en utilisant les solvants préférés que sont l'acétone, l'éther, le chloroforme et leurs mélanges, à une partie en poids de produit à traiter, on ajoute de 5 à 25 parties en volume de solvant puis on agite énergiquement pendant une durée allant de 30 secondes à 10 minutes et à une température comprise entre 0 et 10°C. L'insoluble délipidé ou partiellement délipidé est séparé pr filtration sous vide. En cas de délipidation incomplète, l'insoluble est retraité de la même façon. Pour parvenir au degré de délipidation souhaité, l'opération peut être répétée de 2 à 5 fois.

Ainsi qu'il l'a été décrit précédemment, la méthode de séparation A est essentielle pour préparer les produits de l'invention dont l'activité lipasique spécifique est comprise entre 1 et 100 unités par milligramme de protéine.

Cette méthode A consiste, dans un premier temps, à traiter en milieu aqueux acide les estomacs, tels quels ou facultativement préparés selon les opérations déjà décrites. Ainsi, on pourra utiliser les estom acs entiers, ou leurs parties sélectionnées, ou encore le matériau pulvérilant obtenu après homogénéisation par broyage dans un solvant comme l'acétone ou ce même matériau délipidé.

La seconde opération consiste en un relargage des produits lipasiques provoqué par l'addition de sels hydrosolubles.

D'une manière plus précise, l'opération d'extraction en milieux aqueux acides consiste à agiter très énergiquement les matériaux avec des solutions acides appropriées, puis à séparer la phase liquide qui contient les principes actifs par filtration, centrifugation ou éventuellement décantation.

Ainsi, habituellement, pour un gramme de matériau mis en oeuvre, il est utilisé de 1 à 100 millilitres de solution acide dont le pH est ajusté de 2 à 4.

L'acidité de la phase aqueuse est amenée par des acides de force appropriée pour obtenir de telles valeurs de pH. A cet égard, les acides forts minéraux sont utilisés ou les acides organiques dont le pKa est inférieur à 5. Ce sont essentiellement les acides oxalique, tartrique, malique, citrique, maléique, formique, lactique et acétique ou encore les acides sulfurique, phosphorique et chlorhydrique qui sont les plus couramment utilisés.

Dans le cas où cette opération est effectuée sur les fragments de tissus, l'addition de protéases comme par exemple des pepsines peut améliorer l'extraction de la lipase en milieu aqueux acide.

Les conditions opératoires sont adaptées afin d'éviter la dénaturation irréversible des principes actifs sensibles, notamment à des températures supérieures à 25°C. Généralement, les extractions sont réalisées par mélange énergique des constituants durant une période de 1 minute à 15 heures selon l'efficacité du mélangeur utilisé et à une température comprise entre 0 et 20°C.

D'une façon préférée, pour 1 g de matériau à traiter, il est utilisé de 3 à 60 millilitres de solution d'acide chlorhydrique de pH ajusté de 2 à 4 ± 0,1 solutions réalisées par exemple pour pH 2 en ajoutant 2,0 ml d'acide chlorhydrique à 37 % dans 2 litres d'eau.

Le mélange ainsi obtenu est homogénéisé sous agitation violente durant 5 à 60 minutes et à une température comprise entre 0 et 20°C. La phase aqueuse est ensuite séparée par filtration, décantation ou centrifugation. Dans ce dernier cas, les conditions les plus appropriées consistent à effectuer la centrifugation durant 15 minutes à 1 heure à des vitesses allant de 3 000 à 12 000 tpm et à des températures de 4 à 20°C. La séparation peut être effectuée également par filtration et dans ce cas, l'opération sera avantageusement accélérée en utilisant des systèmes de filtration sous pression.

Tel que, le mélange ainsi obtenu peut être concentré par ultra filtration, congelé et lyophilisé afin d'obtenir l'extrait sous forme solide, ce dernier pouvant être éventuellement délipidé par les opérations précédemment décrites.

Dans ce cas, le mélange est d'abord congelé par refroidissement de - 20° à - 70°C, températures assurées par des mélanges eau - glycol ou carboglace - acétone pour les températures les plus basses, opérations effectuées dans des récipients adaptés au matériel de l'opération de lyophilisation qui suit, et qui est réalisée sous un vide inférieur à 10⁻¹ millibar, l'eau éliminée par sublimation étant condensée par des pièges à une température inférieure à -50°C et notamment à - 80°C refroidis à l'ammoniac ou au fréon. Le produit déshydraté amorphe obtenu est ensuite pulvérisé à froid comme par exemple par cryobroyage à une température inférieure à 0°C, avant d'être délipidé puis extrait selon la méthode A. Toutes les opérations de lyophilisation suivant l'invention s'effectuent d'une manière analogue.

La seconde opération de cette méthode A consiste à précipiter les substances d'activité lipasique contenues dans les solutions aqueuses acides obtenues lors de l'opération précédente. Cette précipitation est effectuée par relargage qui consiste à aj outer à la solution des sels hydrosolubles. Couramment, ces sels comprennent en tant que cations le magnésium, le potassium, le sodium, l'ammonium, et comme anions des acétate, citrate, phosphate et sulfate. Toutefois, les sels préférés sont des combinaisons d'anions polyvalents, en particulier des phosphate et sulfate. Les cations alcalino-terreux tels que le calcium et le baryum, qui provoquent des dénaturations irréversibles des protéines, sont à proscrire. Le sel préféré est le sulfate d'ammonium.

Dans ce cas, pour 1 litre de solution acide à traiter, on ajoute, sous agitation et à une température comprise entre 0 et 20°C, de 80 à 700 g de sulfate d'ammonium cristallisé. L'agitation est maintenue jusqu'à dissolution pratiquement totale du sel, puis le mélange est abandonné au repos à une température comprise entre 0 et 20°C, de façon à amener un relargage le plus complet possible des substances actives, ce qui nécessite de 15 minutes à 20 heures, à la suite de quoi la solution est éliminée par les moyens habituels tels que décantation, filtration, ou centrifugation.

D'une façon particulièrement favorable, cette opération de relargage est réalisée en ajoutant à une température comprise entre 0 et 10°C de 100 à 600 g de sulfate d'ammonium par litre de solution acide à traiter. La solution abandonnée de 30 minutes à 1 heure à 10°C environ laisse déposer une quantité optimale d'insoluble d'activité lipasique qui est séparée par filtration sous vide de 50 millibars environ ou par centrifugation durant 10 à 45 minutes à une vitesse de 3 000 à 10 000 tpm et à une température comprise entre 4 et 10°C.

Le produit ainsi obtenu contient les substances actives d'autres substances biologiques et divers sels minéraux dont ceux introduits au cours des traitements.

Tel quel, il peut être utilisé dans des opérations industrielles, cependant, pour des utilisations notamment pharmaceutiques, il est nécessaire d'éliminer les sels parasites, ce qui est réalisé par filtration sur membrane (dialyse ou ultrafiltration) sur membrane. Cette dernière méthode est particulièrement utilisée. Elle consiste, parès redissolution de l'insoluble dans de l'eau, à soumettre la solution à une ultrafiltration en maintenant le produit à un volume constant par addition d'eau sur une membrane dont la texture perméable aux molécules de faible poids moléculaire retient les produits et notamment les enzymes de poids moléculaire plus élevé.

Ainsi, l'insoluble obtenu par relargage est dissous dans l'eau, le pH de la solution est ajusté entre 2 et 7 par addition d'acide chlorhydrique dilué, et la solution acide ultrafiltrée par circulation tangentielle sur une membrane généralement dont la sélectivité de coupure est de 5.000 à 10000 daltons pour dessaler. Toutes les opération de dessalage s'effectuent d'une manière analogue dans le procédé suivant l'invention.

Durant la filtration, le débit constant est maintenu le plus rapidement possible, ce qui est assuré au moyen d'une pompe péristaltique. Toutefois la pression du fluide sur la membrane est contrôlée et ne doit pas dépsser 1 bar pour ne pas provoquer son colmatage. Dans ces conditions, une membrane de 200 cm² permet de traiter 2 à 3 litres de filtrat par heure.

Après filtration, le rétentat obtenu contenant les substances de poids moléculaire supérieur à celui de la sélectivité de coupure de la membrane, sera concentré de 2 à 5 fois par ultrafiltration sur l'appareil décrit précédemment avantêtre congelé puis lyophilisé sous vide poussé d'environ 10⁻¹ millibar, selon les techniques et conditions opératoires déjà détaillées.

Afin qu'elles sont précédemment décrites, les opérations précédentes permettent d'obtenir, en tant que produits de l'invention, des extraits d'activité lipasique, caractérisés par une activité spécifique telle que définie ultérieurement de 1 et 100 unités par mg de protéine.

Par une méthode B, ces extraits permettent d'obtenir, au moyen de techniques appropriées, des extraits enrichis et la fraction lipasique pure qui sont également des produits de l'invention.

Cette méthode peut comporter deux opérations de purification qui sont :
- la chromatographie d'échanges d'ions utilisant l'aptitude à l'ionisation de certaines fonctions de composés organiques,
- la filtration sur gel qui permet, à l'aide d'un support approprié, de séparer des composés de poids moléculaire et de taille différents.

Dans le cas où les opérations précédentes n'ont pas compris d'étape de délipidation, la filtration sur gel est préférée.

Quand l'extrait lipasique a été délipidé à une étape précédente, la mise en oeuvre de l'une ou de l'autre de ces opérations permet d'obtenir les extraits lipasiques enrichis, dans ce cas la chromatographie d'échanges d'ions est préférée.

L'obtention de la lipase, quant à elle, nécessite la mise en oeuvre successive des deux opérations qui peuvent être effectuées dans un ordre infifférent.

Ces techniques de purification sont résumées dans ce qui suit :
La chromatographie d'échanges d'ions s'adresse à la purification de substances hydrosolubles comprenant des fonctions ionisables capables d'acquérir dans des milieux appropriés des charges positives et négatives qui permettent leur fixation sur des supports chargés de manière opposée. L'élution des produits fixés au support s'effectue progressivement en fonction de leur force de liaison et permet donc de séparer dans certains cas des produits à partir de mélanges.

Ainsi, les enzymes de structures protéiques portent à la fois des fonctions amines et des fonctions carboxyliques dont l'ensemble au pH isoélectrique de la molécule est de charge nulle. Une acidification des solutions au-dessous de ce pH agit sur les fonctions carboxyliques et charge positivement l'enzyme, inversement une alcalinisation agit sur les fonctions aminées et amène une charge négative du produit.

Ces propriétés amphotères des protéines permettent leur purification par des méthodes chromatographiques d'échanges de cations ou d'anions.

Les supports utilisés pour de telles méthodes sont des substances insolubles dans le milieu d'élution utilisé, et comprenant des fonctions ionisables.

Les principaux types de supports sont des résines, des réseaux tridimensionnels de styrène et polyvinylbenzène, des celluloses substituées, des dérivés de dextrane ou encore des dérivés d'agarose. Parmi ces derniers, la firme Pharmacia propose un support polymère d'agarose fortement réticulé qui permet des séparations particulièrement rapides (marque "Fast Flow^{R}").

Ces différents supports comprennent dans leurs structures des fonctions ionisables comme les fonctions sulfonique, carboxylique, phosphorique, arsenique pour les échanges de cations et des fonctions amine primaires, secondaires ou tertiaires pour les échanges d'anions.

D'une façon préférée, les produits de l'invention ont été purifiés par chromatographie d'échanges de cations sur des colonnes de type "Fast Flow S "Sépharose "^{R} (Pharmacia) de dimensions : diamètre 2,6 mm, hauteur 30 cm, dont les supports portent des radicaux sulfo-propyle et qui sont donc échangeurs de cations, ou sur des colonnes contenant un support identique, mais de contenance plus faible (1 ml) type "Fast Flow mono S^{R}" (Pharmacia).

Les extraits à purifier sont dissous dans des tampons d'un pH compris entre 3 et 5 et notamment de 4. On donne ci-dessous des exemples de tampon.

| | | |
|---|---|---|
| a) | Acétate de sodium | 20mM/litre |
| | Chlorure de sodium | 100mM/Litre |
| b) | Acétate de sodium | 20mM/litre |
| | Acide acétique qsp | pH 4 |

Les solutions d'extraits sont déposées sur les gels préalablement équilibrés avec le tampon utilisé pour la purification.

Celle-ci diffère selon le type de colonne utilisé :
- Avec les colonnes de type "mono S" :
L'extrait est solubilisé dans le tampon b). Les produits sont purifiés par le même tampon progressivement enrichi avec un tampon de nature identique, mais contenant 500 mM de chlorure de sodium par litre, afin d'obtenir un gradient d'élution de pH 4 contenant progressivement de 0 à 500 mM de chlorure de sodium par litre.
La modification progressive de la force ionique permet de séparer, pour une concentration de 250 mM environ par litre de chlorure de sodium, une fraction contenant l'extrait lipasique enrichi.
- Avec les colonnes de type "S Sépharose" :
Le produit à purifier est dissous dans le tampon a) et déposé sur la colonne. On élue par ce même tampon puis avec une solution de composition :

| | |
|---|---|
| Acétate de sodium | 20 mM/litre |
| Chlorure de sodium | 200 mM/Litre |

solution qui permet d'éluer une fraction lipasique enrichie en l'extrait cherché.

Cette méthode de purification appliquée à l'extrait obtenu dans la méthode A après relargage permet d'obtenir des fractions contenant l'extrait lipasique enrichi dont l'activité spécifique est comprise entre 100 et 500 unités par milligramme de protéine.

Les fractions aqueuses obtenues par ces purifications peuvent être :
- soit engagées dans une méthode de purification complémentaire, pour obtenir la lipase,
- soit être dessalées par dialyse ou ultrafiltration, puis congelées et lyophilisées par les méthodes déjà décrites afin d'obtenir un extrait lipasique enrichi sous forme solide amorphe.

La filtration sur gel dite de type "tamis moléculaire" permet une séparation des molécules en fonction de leur taille, c'est-à-dire de leur volume ou leur encombrement. Les gels insolubles utilisés sont formés de réseaux tridimensionnels variables accessibles à une tailles de molécule adaptée.

Ainsi, avec un mélange de molécules de tailles différentes, seuls les produits de taille adaptée au réseau du gel seront retenus, les molécules de taille supérieure ne seront pas retenues et seront éluées rapidement. Pour les molécules retenues sur le gel, leur temps de rétention dépend également de leur taille et l'élution de ces produits s'effectue dans un ordre proportionnel à celle-ci, les molécules plus petites étant plus retenues.

Les gels qui possèdent ces propriétés de séparation peuvent être divers et on peut citer :
- les Séphadex^{R} G qui sont constitués de dextranes pontés avec l'épichlorhydrine,
- les Biogels^{R} P qui sont des chaînes de polyacrylamide pontées par du N, Nʹ - méthylène bisacrylamide,
- les Ultrogels ^{R} et les séphacryls^{R} qui combinent une trame mixte de polyacrylamide et d'agarose.

Les produits de la présente invention ont été traités avec de préférence des gels de polyacrylamide de type Sephacryl^{R}.

Les extraits non délipidés sont traités par un tamis dont la limite d'exclusion est supérieure à 1000 000 de daltons.

Ainsi les solutions aqueuses des produits à purifier sont déposées sur une colonne d'une hauteur d'environ 1 mètre contenant de 1 à 7 litres de gel.

L'élution est ensuite effectuée par une solution à pH 6 contenant de 100 à 500 mM de chlorure de sodium et 10 à 30 mM de phosphate disodique. L'éluat est recueilli par fraction et l'activité lipasique recherchée dans ces fractions. Ainsi, les fractions contenant l'activité sont dessalées par dialyse ou par ultrafiltration puis congelées et lyophilisées afin d'obtenir l'extrait lipasique enrichi recherché sous forme solide.

Les extraits lipasiques délipidés sont quant à eux traités par un tamis permettant la séparation de substances d'un poids moléculaire compris entre 30000 et 55000 daltons et notamment entre 45000 et 55000 daltons.

Ainsi, les solutions aqueuses des produits à purifier sont déposées sur une colonne d'une hauteur d'environ un mètre contenant 400 à 500 ml de gel.

L'élution est ensuite effectuée par une solution de pH 6 contenant 200 mM par litre de chlorure de sodium. L'éluat est recueilli par fractions et l'activité lipasique recherchée dans ces fractions.

Ainsi obtenues, les fractions contenant l'activité lipasique peuvent être :
- soit engagés dans une méthode de purification complémentaire pour obtenir la lipase.
- soit être dessalées par dialyse ou par ultrafiltration, puis congelées et lyophilisées afin d'obtenir un extrait lipasique enrichi sous forme solide, pouvant être dans certain cas soumis à délipidation.

Le procédé suivant l'invention a été appliqué à des estomacs de veau, de mouton, de porc, de poulet, de lapin et de cheval. Or, d'une manière surprenante et contrairement, par exemple, à ce que l'on obtient en partant d'estomacs de porc, les substances lipasiques et lipases obtenues à partir d'estomacs de lapin et de cheval ont un maximum d'activité lipasique à un pH compris entre 4 et 5, et notamment de 4,5, alors qu'il est de 6 à 7 pour le porc et qu'il est donc peu actif en milieu gastrique.

En outre, et de façon imprévue, on a également constaté que, contrairement à l'extrait obtenu à partir d'estomacs de porc, les extraits d'estomacs de lapin et de cheval conservaient leur activité lipasique après incubation en milieu acide. Ainsi incubées durant 2 heures à 37°C et à pH 2, ces activités ne sont pas altérées alors que celles des estomacs de porc traitées de façon analogue ne peuvent résister à un pH inférieur à 4.

Par ailleurs, les activités des extraits d'estomacs de lapin et de cheval ont été étudiées dans une gamme de pH de 3 à 9. Entre pH 3 et 7, il a été constaté pour les extraits une activité lipasique égale ou supérieure de 40 % à celle de leur activité maximale qui se situe vers pH 4,5. Les protocoles expérimentaux de ces essais sont décrits dans la suite de ce texte.

Ainsi, les propriétés remarquables de résistance à la dénaturation en milieu acide ainsi que la large plage d'activité des lipases des extraits d'estomacs de lapin et de cheval justifient leur utilisation sous forme de médicament comme substitut d'activités lipasiques dans les divers cas de carence pathologique chex l'homme et les animaux.

L'invention a donc pour objet une lipase ayant un poids moléculaire selon la technique de Laemmli de 49000 daltons, dont 9000 correspondent à des sucres et 40000 à une protéine, dont le nombre des types d'acides aminés est le suivant : Asp. Asn : 45 ; Thr : 19 ; Ser : 28 ; Glx : 30, Pro : 29 ; Gly : 25 ; Ala : 28 ; Val : 27 ; Met : 8 ; Ile : 18 ; Leu : 26 ; Tyr : 16 ; Phe : 18 ; Lys : 17 ; His : 7 ; Arg : 10 ; Cys : 9 et Trp : 6, dont la séquence N-terminale est : Lys-Ser-Ala-Pro-Thr-Asn-Pro-Ser-Glü-Glu-Val-Asn-Met-X-Ile-Ser-Glu-Met-Ile-Ser-Tyr-Trp-Gly-Tyr-Pro-Lys-Tyr-Glu-Val-Val, X désignant un acide aminé non déterminés, dont l'activité lipasique spécifique selon la méthode de Gargouri est supérieure à 1000 U/mg de protéine, dont l'activité maximale est obtenue pour une valeur de pH de 4,5 environ, dont l'activité, à des valeurs de pH de 3 et 7, est au moins égale à la moitié de l'activité maximale, dont l'activité se conserve après incubation pendant 2 heures à 37°C et à une valeur de pH de 2 dont l'acide aminé impliqué dans l'activité lipolytique est la cystéine, qui résiste à la pepsine, est dégradée par la chymotrypsine et par la trypsine, dont le pH isoélectrique est entre 5,7 et 7,1 et qui est susceptible d'être préparée par le procédé suivant l'invention, à partir du fundus d'estomac de lapin.

L'invention a également pour objet une lipase, dont l'activité lipasique spécifique selon la méthode de Gargouri est supérieure à 1000 U/mg de protéine, dont l'activité maximale est obtenue pour une valeur de pH de 4,5 environ, dont l'activité, à des valeurs de pH de 3 et 7, est au moins égale à la moitié de l'activité maximale, dont l'eactivité se conserve après incubation pendant 2 heures à 37°C et à une valeur de pH de 2 et qui est susceptible d'être préparée par le procédé suivant l'invention à partir d'estomacs de cheval.

L'invention vise enfin des extraits lipasiques enrichis délipidés ou non, éventuellement en solution aqueuse, ayant une activité lipasique spécifique selon la méthode de Gargouri, comprise entre 100 et 1000 U/mg de protéine, dont l'activité maximale est obtenue pour une valeur de pH de 4,5 environ, dont l'activité, à des valeurs de pH de 3 et 7, est au moins égale à la moitié de l'activité maximale, dont l'activité se conserve après une incubation pendant 2 heures à 37°C et à une valeur de pH de 2 et qui sont susceptibles d'être préparés par le procédé suivant l'invention.

L'invention vise aussi un extrait lipasique délipidé ou non ayant une activité lipasique selon la méthode de Gargouri, comprise entre 1 et 100 U/mg de protéine, dont l'activité maximale est obtenue pour une valeur de pH de 4,5 environ, dont l'activité, à des valeurs de pH de 3 et 7, est au moins égale à la moitié de l'activité maximale, dont l'activité se conserve après incubation pendant 2 heures à 37°C et à une valeur de pH de 2 et qui est susceptible d'être préparé par le procédé suivant l'invention.

Ces extraits résistent à la pepsine, sont dégradés par les trypsines et ont la cystéine comme acide aminé impliqué dans l'activité lipolytique.

Ces produits sont caractérisés en définis par les propriétés habituellement déterminées pour les enzymes et les extraits enzymatiques, à savoir :
a - activité spécifique,
b - acitivé en fonction du pH,
c - résistance de l'activité lipasique après incubation en milieu acide,
d - détermination du poids moléculaire apparent,
e - activité sur le triglycérides,
f - détermination du pH isoélectrique,
g - détermination de la présence d'un acide aminé essentiel impliqué dans l'activité lipolytique,
h - détermination de la résistance aux protéases,
i - détermination de la présence de sucres.

Les méthodes employées pour ces déterminations sont reportées de façon résumée dans ce qui suit.
a. L'activité spécifique est définie comme étant le rapport de l'activité enzymatique et de la quantité de protéines de l'échantillon exprimée en milligrammes. L'activité lipasique est déterminée par la méthode titrimétrique de Y. Gargouri (Thèse de Docteur d'Etat de l'Université d'Aix-Marseille, 1985) dans laquelle le substrat utilisé est la tributyrine. Le dosage consiste à neutraliser l'acide butyrique libéré sous l'action de la lipase par une solution de soude 0,1 N à pH constant de 6 et à une température de 37°C. Dans ces conditions d'essai, l'activité enzymatique correspond au nombre de micromoles d'acide libérées en une minute par l'action du produit soumis à l'essai.
Pratiquement, l'essai consiste à introduire, dans une cellule de titration thermostatée à 37°C :

| | |
|---|---|
| - Tributyrine | 0,50 ml |
| - Solution isotonique d'albumine bovine de sérum et de taurodésoxycholate de sodium | 14,50 ml |
| (composition : albumine bovine de sérum 100 mg, taurodésoxychlolate de sodium 2 mM, soluté isotonique à 9 % de Nacl q.s.p. un litre). | |

Sous agitation électromagnétique et à l'aide d'un titrimètre automatique, le mélange est amené à pH 6 par addition de soude 0,1 N. Après stabilisations du pH à cette valeur, on ajoute, exactement mesurés, de 0,5 à 1 ml d'une solution aqueuse du composé enzymatique à doser. Dans ces conditions expérimentales, la quantité de solution de soude 0,1 N nécessaire pour maintenir le pH à 6 durant 2 minutes permet le calcul de l'activité lipasique telle que définie précédemment.
Les protéines sont déterminées en présence du réactif de Folin Ciocalteu. En présence d'ion cuivrique en milieu alcalin et de ce réactif phospho-molybdotungstique, il se forme un complexe coloré d'intensité proportionnelle à la quantité de protéines (Lowry et al., 1951). La coloration est comparée à celle d'une gamme étalon préparée avec des quantités connues d'albumine bovine de sérum et permet ainsi de déterminer la concentration en protéines de l'échantillons à tester.
b. L'activité en fonction du pH est déterminée dans une gamme de pH 3 à 9 en utilisant une adaptation de la méthode de dosage précédemment décrite.
Ainsi, le mélange de titration est amené à la valeur de pH choisie pour l'étude, soit par ajout d'une solution de soude 0,1 N, soit par ajout d'une solution d'acide chlorhydrique 0,1 N. La solution enzymatique est ajoutée et le pH maintenu durant 2 minutes à cette même valeur de pH.
Le coefficient de dissociation de l'acide butyrique (pKa = 4,75) autorise des dosages directs à pH 6, des dosages avec un facteur correctif jusqu'à pH 5 et des doages dits "en retour" pour des valeurs inférieures à ce pH.
c. Résistance de l'activité lipasique après incubation en milieu acide. Cette étude consiste à déterminer l'activité lipasique après avoir soumis le produit à l'essai à une incubation durant 2 heures au maximum et à 37°C dans des solutions aqueuses de pH variables de 3 à 9.
Une solution aqueuse de l'extrait enzymatique à étudier est amenée à différentes valeurs de pH par addition de solutions de soude ou d'acide chlorhydrique 0,1 N. Ces solutions sont placées dans un bain thermostaté à 37°C. Des prélèvements sont effectués aux temps 0, 30 minutes, 1 heure et 2 heures et sont décrits contenant la tributyrine comme substrat.
Après avoir rapidement ajusté le pH à 6, le dosage de l'activité lipasique est effectué durant deux minutes par ajout de solution de soude 0,1 N pour maintenir la valeur de pH à 6. L'activité lipasique ainsi déterminée est comparée à celle d'un essai témoin du même produit non incubé et directement titré à pH 6 (c'est-à-dire le prélèvement au temps t = 0), ce qui permet d'obtenir un pourcentage d'activité lipasique après incubation dans les conditions de l'expérience.
d. Le poids moléculaire apparent est déterminé par électrophorèse selon la technique de Laemmli (Nature, 1970, 227 : 680-685) qui consiste à traiter l'échantillon par le dodécylsulfate de sodium et à faire migrer dans un gel de polyacrylamide constitué successivement d'un gel à 5 %, puis 12,5 % (p/v). Des protéines de poids moléculaires connus entre 14 000 et 94 000 daltons sont traitées de façon identique. Après visualisation des protéines au bleu de Coomassie, le poids moléculaire apparent de l'échantillon est déterminé par comparaison de sa migration avec celles des protéines de référence.
Cette méthode permet d'obtenir un poids moléculaire de 48 000 daltons pour une lipase purifiée obtenue à partir d'estomacs de lapin (exemple 6).
e. Les activités de la Lipase ou des extraits lipasiques ont été étudiées sur 3 types de substrat, les triglycérides

| | |
|---|---|
| à courte chaine | La Tributyrine (C₄) |
| à chaine moyenne | le Liprocile ^{R}(C₈C₁₀) |
| à chaine longue | l'Intralipide^{R} 30 % huile de soja |

Pratiquement 2 unités sont ajoutées à 0,5 ml de Tributyrine ou 0,5 ml de Liprocile, lipases ou 5 ml d'une solution d'Intralipide à 30 % et 10 unités d'activité lipasique sont incubée dans un tampon NaCl 9 %, CaCl₂ nM et sérum albumine bovine.
L'étude de l'activité en fonction du pH montre que la lipase a une activité maximale à environ pH 5 pour la Tributyrine, à pH 6 environ pour le Liprocile et à pH 4 pour l'intralipide.
En fait, l'activité des produits lipasiques obtenus est déterminée à pH 5 sur les substrats précédemment énumérés. Les activités constatées sur le Liprocile (R) et l'Intralipide (R) sont respectivement comprises entre 50 et 75 % et 25 et 40 % de celle observée sur la Tributyrine.
f. Détermination du pH isoélectrique. Les protéines placées dans un champ électrique et dans une gradient de pH formé par des ampholytes migrent jusqu'à leur pH isoéléctrique. Le gradient est formé par un mélange de polyélectrolytes d'un poids moléculaire égal ou inférieur à 1000, constitué d'acides aliphatiques polyaminés et polycarboxyliques ayant des points isoélectriques variant de 2 à 11.
Pratiquement sur un gel horizontal préformé 2 à 5 µg d'extrait lipasique sont déposées, les protéines soumises à un champ électrique migrant pendant environ 1 heure. La présence de bandes est visualisée par le réactif au Nitrate d'argent.
Le pH de la lipase est compris entre 5,7 et 7,1. La présence de plusieurs bandes dans ce champ de pH est dûe à une hétérogénéité des chaines sucrées liées à la protéine.
g. Détermination de la présence d'un acide aminé essentiel dans l'activité lipolytique. Cette étude consiste à ajouter à de la lipase ou des extraits lipasiques, un réactif ayant la propriété de bloquer l'activité de l'enzyme.
Les réactifs que nous avons utilisés sont le DNTB (acide 5-5' Dithiobis 2 nitrobenzoique et le 4 PDS (4-4ʹ Dithiopyridine) qui, dans certaines conditions de pH, se fixent sur les groupements sulfhydriques libres de la protéine. Ces réactifs, après fixation, libèrent les groupements colorés suivants :
DNTB libère l'acide 5 Thio 3 nitrobenzoique qui peut être dosé à une longueur d'onde de 420 nm.
4 PDS libère le groupement 4 Thiopyridone qui peut être observé et quantifié à la longueur d'onde de 324 nm.
Ainsi dans une cuve de spectrophotomètre de 1 millilitre on incube dans un tampon pH 8 tris HCl 0,25 M la lipase acide et le réactif en excès. A différents temps l'absorbance du composé libéré est mesurée et sa concentration calculée à l'aide de son coefficient d'extinction molaire
(E l_{cm}, M = 13 600 à 420 nM)
(E l_{cm}, M = 19 800 à 324 nM)
dans le même temps un prélèvement est effectué et permet de mesurer l'activité lipolytique résiduelle avec la méthode décrite en a. L'activité lipasique de la lipase purifiée est ainsi totalement inhibée par ces réactifs. Par ailleurs le calcul permet de déterminer qu'un seul groupement sulfydryle libre participe à l'activité lipolytique, groupement sulfydryle appartenant à l'acide aminé cystéine. Les extraits lipasiques traités dans ces mêmes conditions sont également inhibés.
h. Détermination de la résistance aux protéases. Cette étude a été conduite avec la pepsine de porc, enzyme qui est sécrétée dans l'estomac, la chymotrypsine et le trypsine de porcs, enzymes qui sont secrétées dans le duodénum par le pancréas.
Les extraits lipasiques et la lipase sont incubés pendant 2 heures à pH 7,5 pour la chymotrypsine et la trypsine et à pH 2 pour la pepsine à 37°C. Des prélèvements sont effectués régulièrement toutes les 15 minutes et l'activité résiduelle lipasique est déterminée par la méthode décrite en a.
Ainsi 2 mg de pepsine sont mis en présence de 3 mg de lipase ou d'extrait lipasique dans un tampon pH 2. De la même manière, 2 mg de trypsine ou de chymotrypsine sont mis en présence de 3 mg de lipase ou extraits lipasiques dans un tampon pH 7. L'activité qui servira de référence a été déterminée en c.
L'activité initiale reste inchangée pendant 2 heures avec la pepsine. Au bout de 2 heures, l'activité en présence de chymotrypsine est réduite de 50 % et la trypsine a complètement détruit l'activité lipasique en 2 heures.
i. Détermination de la présence de sucres. Cette étude consiste à hydrolyser les chaines sucrées liées à la protéine par une endogycosidase spécifique. L'ENDOF ^{R} enzyme qui coupe entre deux N acétylglucosamines.

La méthode consiste à déterminer le poids moléculaire apparent du produit obtenu à partir de la lipase traitée par cet enzyme.

Après hydrolyse et traitement selon la méthode décrite en d, il est observé une bande d'intensité majeure qui correspond à un poids moléculaire inférieur à celui obtenu à partir de l'enzyme natif et voisin de 40 000 Daltons.

Par ailleurs, la composition en acides aminés permet, à partir du poids de chaque aminé de déterminer un poids de la protéine d'environ 40 000. La différence avec le poids déterminé par la technique de Laemmli décrite en d, est d'environ 9 000 Daltons et correspond à la fraction sucrée de l'enzyme.

La lipase purifiée a permis de déterminer les caractéristiques biochimiques de la lipase :
- composition en acides aminés,
- séquence N-terminale.

La composition en acides aminés a été déterminée après hydrolyse de la protéine par l'acide chlorhydrique aux temps de 24, 38 et 72 heures. Les hydrolysats sont ensuite analysés suivant la méthode de Spackman D.H. ; Stein W.H. et Moore S. (Anal. Chem. 1957, 228, 999) avec un analyseur automatique d'acides aminés (Spinco-Beckman), les résultats analytiques étant ensuite traités par la méthode de Delaage M. (Biochem. Biophys. Acta, 1968, 168, 573).

Les résidus cystéine sont déterminés par oxydation performique et les tryptophane par hydrolyse en présence d'acide mercaptoéthanesulfonique selon la méthode de Penke B. Ferenzi R., Fouacs K. (Analytical Biochemistry, 1974, 60, 45-50).

Le nombre des deivers types d'acides aminés déterminés est le suivant :
Asp. Asn-45 ; Thr-19 ; Ser-28 ; Glx-30 ; Pro-29 ; Gly-25; Ala-28 ; Val-27 ; Met-8 ; Ile 18 ; Leu-26 ; Tyr-16 ; Phe-18 ; Lys-17 ; His-7 ; Arg-10 ; Cys-9 ; Trp-6.

La séquence N-terminale de la protéine a été déterminée selon la méthode de Fred S. Esch (Anal.Biochem. 1984, 136, pages 39-47). La séquence terminale de 30 acides aminés est la suivante :
Lys- Ser - Ala - Pro- Thr - Asn - Pro - Glu - Val - Asn - Met - X - Ile - Ser - Glu - Met - Ile - Ser - Tyr - Trp - Gly - Tyr - Pro - Ser - Glu - Lys - Tyr - Glu - Val - Val. (X : non déterminé).

L'invention vise enfin un médicament, pour lutter contre les malabsorptions des matières grasses chez l'homme et chez l'animal, qui comprend, à titre de principe actif, une lipase et/ou un extrait lipasique, suivant l'invention.

Les exemples suivants illustrent l'invention.

### EXEMPLE 1

### Extrait lipasique non délipidé préparé suivant la méthode A :

Les estomacs de lapins âgés de 2 à 3 mois sont collectés à l'abattoir. Le tiers supérieur du Fundus est découpé et vidé de son contenu. Les tissus sont congelés à - 20°C puis fragmentés en morceaux inférieurs à 2 cm.

### Homogénéisation et extraction en milieu acide suivant la méthode A :

160 g de fissu fragmentés sont ajoutés à 600 ml de solution chlorhydrique de pH 2,5 à une température de 10 à 12°C contenant 0.06 % p/p de pepsine.

L'homogénéisation est réalisée à une température voisine de 20°C par agitation durant 45 minutes à environ 900 tours/minute du mélange au moyen d'un agitateur hélicoïdale.

L'insoluble est séparé par centrifugation à 5000 tours pendant 30 minutes. La phase acide surnageante jaune pâle est séparée :

| | |
|---|---|
| - Volume | 540 ml |
| - Activité spécifique | |
| - Activité totale | 175000 unités |

### Précipitation au sulfate d'ammonium :

Sous agitation à une température de 12°C, on ajoute peu à peu 147 g de sulfate d'ammonium dans la solution acide obtenue lors de l'opération précédente. Après addition, la solution est abandonnée à 15°C pendant 15 minutes, le précipité relargé est séparé par centrifugation à 3500 tours pendant 20 minutes.

Le culot qui contient la fraction enzymatique est dissous dans 175 ml de tampon phosphate à pH 6. On obtient une solution limpide jaune pâle contenant quelques impuretés mécaniques.

| | |
|---|---|
| - Volume | 175 ml |
| - Activité spécifique | |
| - Activité totale | 124 250 unités |

### EXEMPLE 2

### Extrait lipasique enrichi non délipidé préparé suivant la méthode B :

La solution précédente est filtrée sur des filtres de porosité de 5 µn.

La solution ainsi obtenue est purifiée par chromatographie sur gel sepharyl S 300^{R}, Fournisseur Pharmacia (hauteur 1 mètre, volume 7.51 litres) en éluant avec le tampon pH 6 déjà décrit, à un débit de 115ml/h. La présence de l'activité lipasique recherchée est détectée dès les premières fonctions recueillies. Ces fractions sont recueillies pour obtenir une fraction homogène :

| | |
|---|---|
| - Volume | 830 ml |
| - Activité lipasique spécifique | |
| - Activité lipasique totale | 100 642 unités |

Cette solution dessalée, concentrée, et lyophilisée permet d'obtenir 317 grammes de poudre amorphe jaune claire d'extrait non délipidé d'activité enzymatique de 98000 unités et d'une activité spécifique de 31 unités par mg de poudre

| Activité aux différents pH | |
|---|---|
| pH 3 | 4503 u |
| pH 5 | 8750 u |
| pH 7 | 4412 u |

| Résistance aux pH acides | | |
|---|---|---|
| pH | au temps : 0 | 8300 unités |
| | au temps : 2 heures | 8000 unités |

| Activités sur les Triglycérides à chaines | |
|---|---|
| courtes | 8750 unités |
| moyenne | 6829 unités |
| longue | 3233 unités |

Inhibition de l'activité par les réactifs spécifiques des cystéines
Inhibition complète de l'activité lipasique à pH 8 par le réactif DNTB.
Action des protéases

### Dégradation :

- nulle par la pepsine
- entre 40 et 50 % par la chymotrypsine
- inhibition complète par la trypsine dans les conditions opérato!res précédemment décrites.

Taux de lipide

### EXEMPLE 3

### Extrait lipasique délipidé préparé selon la méthode A :

### a. Préparation

Les parties gastriques de viscères de lapins d'espèce "Janny" ou d'espèce campagnarde, âgés de 2 ou 3 mois, sont collectées à l'aboattoir. Les estomacs sont préparés en éliminant les éventuelles souillures ainsi que les portions d'oesophage, d'intestin et de rat.

### Homogénéisation, déshydratation, délipidation :

225 g de tissus sont broyés à 5°C dans 2250 ml d'acétone dans un appareil antidéflagrant durant 30 secondes et à 30 000 tours par minute. L'insoluble est filtré sous vide sur buchner et lavé par 100 ml d'acétone froid.

L'insoluble est repris et l'opération renouvelée dans les mêmes conditions avec, successivement, les solvants de délipidation suivants : acétone (3 fois), chloroforme (3 fois), éther diéthylique (3 fois).

La poudre est finalement séchée sous vide dans un dessicateur jusqu'à poids constant : poids = 45 g.

### b. Méthode A

### Extraction aqueuse acide

La poudre délipidée est introduite dans 1125 ml de solution chlorhydrique de pH 2,5 préalablement refroidie à 5°C.

Le mélange est agité à 4°C durant 30 minutes puis la fraction insoluble séparée par centrifugation à 10 000 tpm durant 30 minutes. La phase aqueuse est séparée.

| | |
|---|---|
| - Volume | 1125 ml |
| - Activité lipasique spécifique | 7 U/mg de protéine |
| - Activité lipasique totale | 75 000 U |

Activité inchangée après incubation durant 2 heures à 37°C et à pH 2.

### Précipitation au sulfate d'ammonium

Sous agitation, à une température de 4°C, on ajoute peu à peu 352 g de sulfate d'ammonium dans la solution acide obtenue lors de l'opération précédente. Après addition, le mélange est abandonné 30 minutes à la même température, puis centrifugé à 10 000 tpm durant 30 minutes.

Le surnageant est décanté et le culot de centrifugation qui contient la fraction enzymatique est dissous dans 300 ml d'eau. Les caractéristiques enzymatiques de la solution sont déterminées :

| | |
|---|---|
| - Activité lipasique spécifique | 73 U/mg de protéine |
| - Activité lipasique totale | 37 500 U |

Cette solution est directement engagée pour purification selon la méthode B dans l'exemple 4 qui suit, en ayant été soumise au préalable au traitement suivant :
Le sulfate d'ammonium contenu dans la solution est éliminé par dialyse à 4°C et sous agitation contre 15 litres de solution tampon à pH 4 (acétate de sodium 20 mM/l, chlorure de sodium 100 mM/l, acide acétique q.s. pH 4). Une heure après, le tampon est éliminé et l'opération répétée à deux reprises, en renouvelant la solution tampon. On obtient finalement 300 ml de solution d'activité lipasique dans laquelle le sulfate d'ammonium est éliminé. Cette solution est purifiée comme il est décrit à l'exemple qui suit.

### EXEMPLE 4

### Extrait lipasique enrichi préparé selon la méthode B:

### Chromatographie sur échangeur d'ions :

La purification est effectuée sur une colonne type "Fast Flow S Sepharose" (fournisseur Pharmacia) contenant 60 ml de gel (diamètre 26 mm).

La colonne est équilibrée avec la solution tampon de pH 4 déjà utilisée pour la dialyse, à raison d'un débit de 100 ml par heure. Les 300 ml de solution sont ensuite introduits et l'élution poursuivie dans les mêmes conditions avec la solution de pH 4 puis une seconde solution de composition acétate de sodium 20 mM/l, chlorure de sodium 200 mM/l de pH 6,5.

Après 480 ml d'élution, on recueille 40 ml d'éluat contenant l'activité lipasique dont les caractéristiques sont :

| | |
|---|---|
| - Activité lipasique spécifique | 350 U/mg de protéine |
| - Activité lipasique totale | 28 000 U. |

La solution, congelée puis lyophilisée, permet d'obtenir 2,8 g d'extrait enrichi d'acitivité lipasique spécifique de 10 000 U/gramme de produit.

### EXEMPLE 5

### Extrait lipasique préparé selon la méthode A :

### a. Préparation

50 g de tissu identique à celui de l'exemple 2 sont homogénéisés, déshydratés et partiellement délipidés à l'acétone selon la technique de ce même exemple.

Après élimination de l'eau et de l'acétone résiduels, sous un vide de 40 millibars et à 25°C, on obtient 10 g de produit qui est traité selon la méthode A.

### b. Méthode A

Egalement appliquée selon l'exemple 2.

| Extraction aqueuse acide | |
|---|---|
| - Volume obtenu | 222 ml |
| - Activité lipasique spécifique | 2,6 U/mg protéine |
| - Activité lipasique totale | 8850 U. |

### Précipitation au sulfate d'ammonium

Aprés centrifugation, le culot est repris par 19 ml de solution tampon (chlorure de sodium 150 millimoles/litre, pH ajusté à 3 par acide acétique).

| | |
|---|---|
| - Activité lipasique spécifique | 17 U/mg protéine |
| - Activité lipasique totale | 6550 U. |

La solution engagée telle quelle dans l'opération de purification est décrite dans l'exemple 6.

### EXEMPLE 6

### Lipase pure préparée selon la méthode B :

### Filtration sur tamis moléculaire

La solution obtenue à l'exemple précédent est purifiée sur une solution de gel Sephacryl S 200 (fournisseur Pharmacia) (diamètre 26 cm - hauteur 1 m) en utilisant pour l'élution le tampon de pH 3 déjà décrit à un débit de 16 ml par heure.

Après 300 ml d'éluat, on obtient dans un volume de 50 ml la solution contenant l'activité lipasique purifiée.

| | |
|---|---|
| - Activité lipasique spécifique | 257 U/mg de protéine |
| - Activité lipasique totale | 4550 U. |

### Chromatographie sur échangeur d'ions

L'opération est réalisée sur une colonne "mono S Fast Flow" (fournisseur Pharmacia) d'un volume de 0,98 ml. Les 50 ml de solution obtenus précédemment sont déposés sur la colonne puis l'élution effectuée à raison d'un débit de 1 ml par minute, avec une solution tampon de pH 4 (acétate de sodium 20 mmoles/litre comprenant un gradient linéaire en chlorure de sodium compris entre 0 et 500 mmoles par litre).

La lipase est éluée à une concentration de 250 mmo les par litre en chlorure de sodium dans un volume de 20 ml.

Les caractéristiques du produit obtenu sont les suivantes :

| | |
|---|---|
| - Poids total de protéines | 3,4 mg |
| - Activité lipasique spécifique | 1067 U/mg de protéine |
| - Activité lipasique totale | 3630 U |
| - Poids moléculaire apparent (technique de Laemmli) | 48000 |

| Activité aux différents pH | |
|---|---|
| pH 3 | 100 unités |
| pH 5 | 170 unités |
| pH 7 | 85 unités |

| Résistance aux pH acides | | |
|---|---|---|
| pH 2 | au temps : O | 150 unités |
| | au temps : 2 heures | 145 unités |

### Poids moléculaire

On observe une bande à PM = 49 000

| Activités sur les Triglycérides à chaines | |
|---|---|
| courte | 1200 unités |
| moyenne | 760 unités |
| longue | 300 unités |

### Détermination du pH isoélectrique

12 bandes comprises entre pH 5,7 et 7,1
Inhibition de l'activité par les réactifs spécifiques des cystéines
DNTB : innibition en 240 minutes et une molécule de DNTB inhibe une molécule de lipase.
4PDS : inhibition en 60 minutes et une molécule de 4PDS inhibe une molécule de lipase.

### Action des protéases

Dégradation
- nulle par la pepsine
- entre 40 et 50 % par la chymotrypsine
- inhibition complète par la trypsine dans les conditions opératoires précédemment décrites.

### Détérmination de la présence de sucres

La lipase native déposée sur un gel électrophorétique montre une bande de PM : 49 000. Après incubation avec l'endo F pendant des temps variables une bande d'intensité croissante en fonction du temps apparaît de poids moléculaire correspondant à 40 000 daltons.

### EXEMPLE 7

### Extrait lipasique préparé selon la méthode A :

La préparation est effectuée à partir de muqueuse gastrique de cheval en faisant directement intervenir la méthode A.

### Extraction aqueuse acide

228 g de muqueuse gastrique de cheval fragmentée en lambeaux de 1 cm² environ sont introduits dans 910 ml d'eau refroidie à 4°C. Le pH du mélange est ajusté à 2,5 ± 0,1 par addition d'acide chlorhydrique concentré à 37 % (p/v). A 4°C l'ensemble est broyé à 30 000 tours par minutes pendant deux périodes de 30 secondes espacées de 2 minutes.

Le broyat est ensuite maintenu sous agitation électromagnétique à froid durant 30 minutes puis la solution acide est séparée par centrifugaiton à 10 000 tpm pendant 30 minutes.

Les 892 ml de solution acide contiennent les substances d'activité lipasique à raison de 15,5 U spécifiques par ml de solution ou 0,74 U spécifique par mg de protéine. Activité spécifique lipasique totale : 13 800 U.

### Précipitation au sulfate d'ammonium

Sous agitation électromagnétique et à 4°C, 500 g de sulfate d'ammonium sont ajoutés à la solution acide précédente. L'agitation est maintenue 30 minutes à cette température puis l'insoluble est séparé par centrifugation à 10 000 tpm durant 30 minutes. Le culot est repris par 50 ml de solution à 9 % en chlorure de sodium et le mélange à nouveau centrifugé dans les mêmes conditions.

Le surnageant contient l'activité lipasique enrichie :

| | |
|---|---|
| - Volume | 51 ml |
| - Activité lipasique spécifique | 192 U/ml |
| | 5,6 U/mg de protéine |
| - Activité lipasique totale | 9800 U |

Cette solution peut être traitée selon les techniques déjà décrites pour obtenir un produit d'état solide.

### EXEMPLE 8

Des estomacs de lapin identiques à ceux utilisés dans l'exemple N^{o} 1 sont utilisés. On sépare l'antre (partie A) du fundus (partie B). Le fundus est lui même segmenté en 4 parties égales coupées dans le sens de la largeur de l'organe. (B₁, B₂, B₃, B₄ classés à partir de l'antre). Ces différents tissus sont traités d'une façon identique à celle de l'exemple N^{o} 1. Les activité lipasiques obtenues dans chaque extrait acide sont déterminées par la méthode de Gargouri. On obtient les résultats suivants :
A = 0
B₁ = 0
B₂ = 0
B₃ = 0
B₄ = 6000 unités

L'activité lipasique se trouve lacalisée dans la partie supérieure du fundus.

Indépendamment d'une absence de toxicité chez l'animal, les produits de l'invention montrenet chez l'homme des effets favorables sur les troubles pathologiques de la digestion.

La recherche de la toxicité a été effectuée sur des rats Wistar de 7 à 8 semaines auxquels il a été administré, par voie orale, une solution aqueuse saturée en extrait lipasique obtenu à partir d'estomacs de lapin à raison de 1 ml contenant 50 unités lipasiques pour 100 g de poids corporel d'animal, et ce quotidiennement durant 4 semaines.

A la fin du traitement, les examens suivants conseillés par l'OCDE ont été pratiqués : sur l'animal vivant, des examens hématologiques, biochimiques sanguins et urinaires ; sur l'animal, après sacrifice, des examens histopathologiques.

Par rapport aux animaux teméoins non traités, il n'a été constaté aucune anomalie chez les animaux ayant reçu l'extrait lipasique. Ces études et la parfaite tolérance des animaux durant le traitement montrent l'absence de toxicité et l'innocuité du produit.

L'efficacité des produits de l'invention, seuls ou associés à d'autres enzymes utilisées dans la pathologie digestive humaine, a également été montrée.

Les produits présentés sous forme de poudre ou de capsules ont été proposés à raison de 300 à 6000 unités lipasiques par jour, selon la gravité de leur état, à des patients âgés de 18 à 75 ans, atteints de pancréatite chronique alcoolique établie. Ces malades, présentant avant traitement une stéatorrhée égale ou supérieure à 10 g par 24 heures, ont reçu les produits par ingestion au cours des repas ou en mélange dans leur nourriture durant 6 mois.

Dans la plupart des cas, il a été constaté une diminution de la stéatorrhée et une action corrective sur la fréquence et le poids des selles. De même, les symptômes d'inconfort, tels que douleurs abdominales et ballonnements ont régressé. Fréquemment, des reprises de poids ont été observées. Aucune intolérance n'a été signalée au cours de cette étude représentative de l'efficacité des produits de l'invention et ce, même comparativement à d'autres enzymes comme les pancréatines, qui n'ont pas montré d'effets aussi significatifs de l'amélioration de l'état général des malades.

Par ailleurs, des enfants atteints de mucoviscidose et dont le coefficient d'absorption des graisses était inférieur à 90 %, ont été traités durant 14 jours avec des doses de produit comparables à celles de l'essai précédent.

Au cours de cette étude, le taux de lipides dans les selles, le poids et le nombre journalier de celles-ci a été contrôlé, ainsi que l'appréciation de l'intensité des douleurs et des ballonnements abdominaux.

On a constaté une nette amélioration de la stéatorrhée et de l'état général. Dans cette étude, des produits de l'invention se sont encore montrés d'efficacité supérieure à celle de la pancréatine.

Les produits de l'invention sont administrés sous fomre de médicament de présentation adaptée à la nature et la gravité de l'affection à traiter. A ce titre, on utilise des comprisés, gélules, capsules molles, granules, microgranules ou des formes pseudo-liquides comme les sirops, les suspensions et les gels.

Les extraits lipasiques peuvent être associés à diverses substances actives sur les symptômes associés au phénomène de malabsorption, ainsi, il est possible d'utiliser des protecteurs gastriques, des antiulcéreux, des anti-reflux gastrooesophagiens ainsi que d'autres enzymes actives dans les phénomènes de la digestion.

Les formes médicamenteuses précédemment décrites sont préparées selon des techniques habituelles à l'homme de l'art et avec divers adjuvants couramment utilisés en pratique pharmaceutique, comme des charges neutres permettant la dilution appropriée du principe actif telles que le lactose, le saccharose , le sorbitol, le mannitol, l'amidon, la cellulose ou des sels comme des phosphates. On utilise également des produits de propriétés désintégrantes, liantes, ou encore lubrifiantes.

Optionnellement, les formes obtenues peuvent être enrobées par du sucre pour obtenir des dragées ou par un film hydrosoluble, ces enrobages ayant pour objet de masquer le goût éventuellement désagréable de la préparation.

Les formes liquides déjà citées, particulièrement utiles en pédiatrie et en gériatrie, sont avantageusement présentées en ajoutant des arômes et des colorants qui amènent des caractères organoleptiques attrayants.

La stabilité des diverses préparations peut être assurée par l'addition de produits antioxydants ou conservateurs tels que le tocophérol, l'acide ascorbique, l'acide sorbique et ses sels, etc...

Pour illustrer les médicaments contenant les produits issus de l'invention, la préparation des comprimés et de poudres est décrite.

### Comprimés

### Formule pour un comprimé de 180 mg dosé à 1000 U lipasiques.

Produit de l'exemple 3 à 73 U/mg de protéine. Quantité suffisante pour 1000 U, soit 100 mg.

| | |
|---|---|
| - Amidon de maïs | 22 mg |
| - Phosphate dicalcique | 30 mg |
| - Cellulose microcristalline | 15 mg |
| - Silice | 12 mg |
| - Stéarate de magnésium | 1 mg |

### Fabrication

Excepté le stéarate de magnésium, les comprimés sont intimement mélangés. Le produit obtenu est compacté, puis calibré sur une grille d'ouverture de maille de 1 mm. Le grain obtenu est lubrifié par addition du stéarate de magnésium, puis comprimé sur machine rotative à raison de 180 mg par unité.

### Poudre

### Formule pour 1 g de poudre dosée à 400 U lipasiques par gramme.

Produit de l'exemple 5 à 17 U/mg de protéine.

| | |
|---|---|
| - Quantité suffisante pour 400 U soit | 0,8215 g |
| - Acide ascorbique | 0,0025 g |
| - Lactose | 0,0760 g |
| - Arôme orange naturel en poudre | 0,1000 g |

### Fabrication

Les constituants sont introduits successivement dans un mélangeur approprié en acier inoxydable. Après homogénéisation, le mélange est réparti en flacons de verre brun qui sont fermés hermétiquement.

Le médicament suivant l'invention comprend donc de 56 à 82 % en poids de principe actif et de 44 à 18 % en poids d'excipient.

Ainsi qu'il a été montrée précédemment, la toxicité des produits de l'invention est insignifiante, ce qui autorise selon la gravité des cas, à traiter à des posologies de 300 jusqu'à plus de 6000 unités lipasiques par jour.

Cette posologie est également adaptée à l'âge du patient et, dans les cas les plus généraux, la posologie journalière est de 600 à 4000 unités lipasiques par jour, qui sont administrées le plus souvent en trois prises au moment des repas.

Les compositions pharmaceutiques contenant les produits de l'invention sont particulièrement appropriées aux traitements des troubles de malabsorption des corps gras, notamment dans les cas de pancréatites de diverses origines : alcooliques, héréditaires, hypercalcémiques ou tropicales.

Elles sont également utiles à la guérison des séquelles d'interventions chirurgicales telles que la gastrectomie et la résection partielle ou totale du pancréas qui peuvent provoquer une stéatorrhé.

Leur indication concerne aussi les cas de mucoviscidose et les carences en lipase, colipase et entérokinase.

Les symptômes qui sont associés à ces affections sont généralement la stéatorrhée, les diarrhées, les douleurs abdominales et des états de dénutrition. Le traitement au moyen des produits de l'invention avec une posologie et une durée appropriées provoque leur régression.

D'une façon générale, les compositions contenant les p roduits de l'invention sont utiles et favorables aux phénomènes d'assimilation des matières grasses alimentaires. Aussi ces produits peuvent-ils être donnés en supplémentation aux sujets normaux afin d'accélérer et/ou de compléter l'assimilation naturelle des graisses et par la même d'accélérer et/ou augmenter leur apport énergétique.

## Revendications

1. Procédé de préparation d'extraits lipasiques, ou d'une lipase, caractérisé en ce qu'il consiste :
a) pour obtenir un extrait lipasique :
- à mettre le fundus d'estomac de lapin adulte âgé d'au moins 2 mois ou de cheval adulte âgé d'au moins 3 ans en contact avec un milieu aqueux acide ayant un pH de 1,5 à 5, à raison de 1 à 10 parties en volume du milieu acide pour 1 partie en poids de fundus, à une température de 4 à 30°C et pendant une minute à 15 heures pour obtenir des matières solides et une solution aqueuse contenant une partie lipasique,
- à séparer la solution aqueuse des matières solides pour obtenir une solution aqueuse séparée,
- à ajouter à la solution aqueuse séparée une quantité suffisante de sel hydrosoluble et à l'y laisser pendant une durée suffisante pour relarguer l'extrait lipasique cherché et pour obtenir une solution surnageante, et
- à séparer l'extrait lipasique cherché de la solution surnageante et à le recueillir.
b) pour obtenir un extrait lipasique dessalé :
- à dissoudre l'extrait lipasique dans une phase aqueuse,
- à filtrer la phase aqueuse sur une membrane ayant un seuil de coupure compris entre 5000 et 10000 daltons pour obtenir une solution de l'extrait lipasique dessalé, et
- à lyophiliser la solution d'extrait lipasique dessalé en l'extrait lipasique dessalé.
c) pour obtenir un extrait lipasique délipidé
- à effectuer en outre sur l'une quelconque des parties solides contenant l'extrait lipasique que sont le fundus d'estomac, l'extrait lipasique et l'extrait lipasique dessalé une opération de délipidation.
d) pour obtenir un extrait lipasique enrichi non délipidé :
- à dissoudre l'extrait lipasique dans un tampon de pH compris entre 2 et 7, pour obtenir une solution tamponnée,
- à chromatographier la solution tamponnée de pH compris entre 2 et 7 sur un tamis moléculaire dont la limite d'exclusion est supérieure à 1.000.000 daltons et à recueillir la fraction d'élution exclue contenant l'extrait lipasique enrichi non délipidé en solution tamponnée,
- à filtrer cette fraction d'élution exclue sur une membrane ayant un seuil de coupure de 10.000 daltons pour obtenir une fraction dessalée, et
- à lyophiliser cette fraction dessalée en l'extrait lipasique enrichi non délipidé cherché.
e) pour obtenir un extrait lipasique enrichi, délipidé :
e1
- à dissoudre l'extrait lipasique dans de l'eau pour obtenir une solution aqueuse contenant l'extrait lipasique,
- à filtrer la solution aqueuse contenant l'extrait lipasique sur une membrane ayant un seuil de coupure compris entre 5000 et 10000 daltons pour obtenir une solution exempte de sel,
- à adsorber la solution exempte de sel sur un support échangeur d'ions,
- à désorber du support par un éluant dont on augmente la force ionique progressivement en fonction du temps,
- à recueillir une fraction d'élution ayant une activité lipasique et contenant l'extrait lipasique enrichi délipidé,
- à filtrer la fraction d'élution ayant une activité lipasique sur une membrane ayant un seuil de coupure compris entre 5000 et 10000 daltons pour obtenir une fraction d'élution dessalée, et
- à lyophiliser cette fraction d'élution dessalée en l'extrait lipasique enrichi délipidé cherché, ou
e2
- à chromatographier la solution tamponnée de pH compris entre 2 et 7 obtenue sous d ci-dessus sur un tamis moléculaire,
- à recueillir une fraction d'élution retenue correspondant à des masses moléculaires comprises entre 30000 et 55000 daltons et contenant l'extrait lipasique enrichi délipidé,
- à filtrer cette fraction d'élution retenue correspondant à des masses moléculaires comprises entre 30000 et 55000 et contenant l'extrait lipasique enrichi sur une membrane ayant un seuil de coupure compris entre 5000 et 10000 daltons pour obtenir une solution de l'extrait lipasique enrichi délipidé dessalé, et
- à lyophiliser cette solution de l'extrait lipasique enrichi délipidé dessalé en l'extrait lipasique enrichi délipidé cherché.
f) pour obtenir la lipase :
f1
- à partir de la fraction d'élution ayant une activité lipasique et contenant l'extrait lipasique enrichi délipidé obtenu en e1 ci-dessus,
- à la chromatographier sur un tamis moléculaire,
- à recueillir une fraction d'élution retenue correspondant à des masses moléculaires comprises entre 45000 et 55000 daltons et contenant la lipase,
- à filtrer cette fraction d'élution contenant la lipase sur une membrane ayant un seuil de coupure de 5000 à 10000 daltons pour obtenir une solution dessalée contenant la lipase, et
- à lyophiliser cette solution dessalée contenant la lipase, ou
f2
- à partir de la fraction d'élution retenue correspondant à des masses moléculaires comprises entre 30000 et 55000 daltons et contenant l'extrait lipasique enrichi délipidé obtenu en e2 ci-dessus,
- à l'adsorber sur un support échangeur d'ions,
- à désorber du support par un éluant dont on augmente la force ionique progressivement en fonction du temps,
- à recueillir une fraction d'élution ayant une activité lipasique et contenant la lipase,
- à filtrer la fraction d'élution ayant une activité lipasique et contenant la lipase sur une membrane ayant un seuil de coupure de 5000 à 10000 daltons pour obtenir une fraction d'élution dessalée contenant la lipase, et
- à lyophiliser cette fraction d'élution dessalée contenant la lipase en la lipase cherchée.

2. Procédé suivant la revendication 1, caractérisé en ce qu'il consiste à effectuer la mise en contact à une température comprise entre 0 et 20°C.

3. Procédé suivant la revendication 1 ou 2, caractérisé en ce qu'il consiste à séparer la solution aqueuse des matières solides par filtration, par décantation ou par centrifugation à une température inférieure à 25°C et, de préférence, à une température comprise entre 4 et 20°C.

4. Procédé suivant l'une des revendications 1 à 3, caractérisé en ce qu'il consiste à ajouter de 80 à 700 grammes de sels hydrosolubles et à les y laisser pendant 15 minutes à 20 heures à une température comprise entre 0 et 20°C.

5. Procédé suivant la revendication 4, caractérisé en ce que les sels sont des sels de magnésium, de potassium, de sodium et, de préférence, d'ammonium d'anions polyvalents, notamment de phosphate ou de sulfate.

6. Procédé suivant l'une des revendications précédentes, caractérisé en ce qu'il consiste à effectuer l'opération de délipidation en mettant les parties solides en contact avec des solvants aptes à solubiliser les matières grasses comme des hydrocarbures, des éthers, des cétones, des alcools ou des halogénures de carbone ayant un point d'ébullition inférieur à 80°C.

7. Procédé suivant l'une des revendications précédentes, caractérisé en ce qu'il consiste à effectuer les adsorptions sur support échangeur d'ions sur des supports échangeurs de cations.

8. Procédé suivant l'une des revendications précédentes, caractérisé en ce qu'il consiste à faire précéder la mise en contact avec le milieu aqueux acide d'une fragmentation du fundus à une dimension inférieure à 1 cm.

9. Une lipase ayant un poids moléculaire selon la technique de Laemmli de 49 000 daltons dont 9000 correspondent à des sucres et 40000 à une protéine, dont le nombre des types d'acides aminés est le suivant :
Asp. Asn : 45; Thr : 19; Ser : 28; Glx : 30; Pro : 29; Gly : 25; Ala : 28; Val : 27; Met : 8; Ile : 18; Leu : 26; Tyr : 16, Phe : 18 : Lys : 17; His : 7; Arg : 10; Cys : 9 et Trp : 6, dont la séquence N-terminale est :
Lys - Ser - Ala - Pro - Thr - Asn - Pro - Glu - Val - Asn - Met - X - Ile - Ser - Glu - Met - Ile - Ser - Tyr - Trp - Gly - Tyr - Pro - Ser - Glu - Lys - Tyr - Glu - Val - Val- X désignant un acide aminé non déterminé, dont l'activité lipasique spécifique selon la méthode de Gargouri est supérieure à 1000 U/mg de protéine, dont l'activité maximale est obtenue pour une valeur de pH de 4,5 environ, dont l'activité à des valeurs de pH de 3 et 7, est au moins égale à la moitié de l'activité maximale, dont l'activité se conserve après incubation pendant 2 heures, à 37°C et à une valeur de pH de 2,dont l'acide aminé impliqué dans l'activité lipolytique est la cystéine, qui résiste à la pepsine, est dégradée par la chymotrypsine et par la trypsine, dont le pH isoélectrique est entre 5,7 et 7,1 et qui est susceptible d'être préparée par le procédé suivant les revendications 1 à 8 à partir de fundus de l'estomac de lapin.

10. Médicament pour lutter contre les malabsorptions des matières grasses chez l'homme et chez l'animal, caractérisé en ce qu'il comprend, à titre de principe actif, une lipase et/ou un extrait lipasique suivant la revendication 9

## Revendications (Revendications pour l'(les) Etat(s) contractant(s) suivant(s): AT, ES)

1. Procédé de préparation d'extraits lipasiques, ou d'une lipase, caractérisé en ce qu'il consiste :
a) pour obtenir un extrait lipasique :
- à mettre le fundus d'estomac de lapin adulte âgé d'au moins 2 mois ou de cheval adulte âgé d'au moins 3 ans en contact avec un milieu aqueux acide ayant un pH de 1,5 à 5, à raison de 1 à 10 parties en volume du milieu acide pour 1 partie en poids de fundus, à une température de 4 à 30°C et pendant une minute à 15 heures pour obtenir des matières solides et une solution aqueuse contenant une partie lipasique,
- à séparer la solution aqueuse des matières solides pour obtenir une solution aqueuse séparée,
- à ajouter à la solution aqueuse séparée une quantité suffisante de sel hydrosoluble et à l'y laisser pendant une durée suffisante pour relarguer l'extrait lipasique cherché et pour obtenir une solution surnageante, et
- à séparer l'extrait lipasique cherché de la solution surnageante et à le recueillir.
b) pour obtenir un extrait lipasique dessalé :
- à dissoudre l'extrait lipasique dans une phase aqueuse,
- à filtrer la phase aqueuse sur une membrane ayant un seuil de coupure compris entre 5000 et 10000 daltons pour obtenir une solution de l'extrait lipasique dessalé, et
- à lyophiliser la solution d'extrait lipasique dessalé en l'extrait lipasique dessalé.
c) pour obtenir un extrait lipasique délipidé
- à effectuer en outre sur l'une quelconque des parties solides contenant l'extrait lipasique que sont le fundus d'estomac, l'extrait lipasique et l'extrait lipasique dessalé une opération de délipidation.
d) pour obtenir un extrait lipasique enrichi non délipidé :
- à dissoudre l'extrait lipasique dans un tampon de pH compris entre 2 et 7, pour obtenir une solution tamponnée,
- à chromatographier la solution tamponnée de pH compris entre 2 et 7 sur un tamis moléculaire dont la limite d'exclusion est supérieure à 1.000.000 daltons et à recueillir la fraction d'élution exclue contenant l'extrait lipasique enrichi non délipidé en solution tamponnée,
- à filtrer cette fraction d'élution exclue sur une membrane ayant un seuil de coupure de 10.000 daltons pour obtenir une fraction dessalée, et
- à lyophiliser cette fraction dessalée en l'extrait lipasique enrichi non délipidé cherché.
e) pour obtenir un extrait lipasique enrichi, délipidé :
e1
- à dissoudre l'extrait lipasique dans de l'eau pour obtenir une solution aqueuse contenant l'extrait lipasique,
- à filtrer la solution aqueuse contenant l'extrait lipasique sur une membrane ayant un seuil de coupure compris entre 5000 et 10000 daltons pour obtenir une solution exempte de sel,
- à adsorber la solution exempte de sel sur un support échangeur d'ions,
- à désorber du support par un éluant dont on augmente la force ionique progressivement en fonction du temps,
- à recueillir une fraction d'élution ayant une activité lipasique et contenant l'extrait lipasique enrichi délipidé,
- à filtrer la fraction d'élution ayant une activité lipasique sur une membrane ayant un seuil de coupure compris entre 5000 et 10000 daltons pour obtenir une fraction d'élution dessalée, et
- à lyophiliser cette fraction d'élution dessalée en l'extrait lipasique enrichi délipidé cherché, ou
e2
- à chromatographier la solution tamponnée de pH compris entre 2 et 7 obtenue sous d ci-dessus sur un tamis moléculaire,
- à recueillir une fraction d'élution retenue correspondant à des masses moléculaires comprises entre 30000 et 55000 daltons et contenant l'extrait lipasique enrichi délipidé,
- à filtrer cette fraction d'élution retenue correspondant à des masses moléculaires comprises entre 30000 et 55000 et contenant l'extrait lipasique enrichi sur une membrane ayant un seuil de coupure compris entre 5000 et 10000 daltons pour obtenir une solution de l'extrait lipasique enrichi délipidé dessalé, et
- à lyophiliser cette solution de l'extrait lipasique enrichi délipidé dessalé en l'extrait lipasique enrichi délipidé cherché.
f) pour obtenir la lipase :
f1
- à partir de la fraction d'élution ayant une activité lipasique et contenant l'extrait lipasique enrichi délipidé obtenu en e1 ci-dessus,
- à la chromatographier sur un tamis moléculaire,
- à recueillir une fraction d'élution retenue correspondant à des masses moléculaires comprises entre 45000 et 55000 daltons et contenant la lipase,
- à filtrer cette fraction d'élution contenant la lipase sur une membrane ayant un seuil de coupure de 5000 à 10000 daltons pour obtenir une solution dessalée contenant la lipase, et
- à lyophiliser cette solution dessalée contenant la lipase, ou
f2
- à partir de la fraction d'élution retenue correspondant à des masses moléculaires comprises entre 30000 et 55000 daltons et contenant l'extrait lipasique enrichi délipidé obtenu en e2 ci-dessus,
- à l'adsorber sur un support échangeur d'ions,
- à désorber du support par un éluant dont on augmente la force ionique progressivement en fonction du temps,
- à recueillir une fraction d'élution ayant une activité lipasique et contenant la lipase,
- à filtrer la fraction d'élution ayant une activité lipasique et contenant la lipase sur une membrane ayant un seuil de coupure de 5000 à 10000 daltons pour obtenir une fraction d'élution dessalée contenant la lipase, et
- à lyophiliser cette fraction d'élution dessalée contenant la lipase en la lipase cherchée.

2. Procédé suivant la revendication 1, caractérisé en ce qu'il consiste à effectuer la mise en contact à une température comprise entre 0 et 20°C.

3. Procédé suivant la revendication 1 ou 2, caractérisé en ce qu'il consiste à séparer la solution aqueuse des matières solides par filtration, par décantation ou par centrifugation à une température inférieure à 25°C et, de préférence, à une température comprise entre 4 et 20°C.

4. Procédé suivant l'une des revendications 1 à 3, caractérisé en ce qu'il consiste à ajouter de 80 à 700 grammes de sels hydrosolubles et à les y laisser pendant 15 minutes à 20 heures à une température comprise entre 0 et 20°C.

5. Procédé suivant la revendication 4, caractérisé en ce que les sels sont des sels de magnésium, de potassium, de sodium et, de préférence, d'ammonium d'anions polyvalents, notamment de phosphate ou de sulfate.

6. Procédé suivant l'une des revendications précédentes, caractérisé en ce qu'il consiste à effecteur l'opération de délipidation en mettant les parties solides en contact avec des solvants aptes à solubiliser les matières grasses comme des hydrocarbures, des éthers, des cétones, des alcools ou des halogénures de carbone ayant un point d'ébullition inférieur à 80°C.

7. Procédé suivant l'une des revendications précédentes, caractérisé en ce qu'il consiste à effectuer les adsorptions sur support échangeur d'ions sur des supports échangeurs de cations.

8. Procédé suivant l'une des revendications précédentes, caractérisé en ce qu'il consiste à faire précéder la mise en contact avec le milieu aqueux acide d'une fragmentation du fundus à une dimension inférieure à 1 cm.

9. L'utilisation de la lipase et/ou d'un extrait lipasique tel que préparé à la revendication 1 pour l'obtention d'un médicament destiné à lutter contre les malabsorption des matières grasses chez l'homme et chez l'animal.

10. Procédé de préparation d'un médicament pour lutter contre les malabsorption des graisses chez l'homme et chez l'animal, caractérisé en ce qu'il consiste à mélanger la lipase et/ou un extrait lipasique tel que préparé à la revendication 1 à un véhicule ou excipient pharmaceutique.

## Revendications (Revendications pour l'(les) Etat(s) contractant(s) suivant(s): GR)

1. Procédé de préparation d'extraits lipasiques, ou d'une lipase, caractérisé en ce qu'il consiste :
a) pour obtenir un extrait lipasique :
- à mettre le fundus d'estomac de lapin adulte âgé d'au moins 2 mois ou de cheval adulte âgé d'au moins 3 ans en contact avec un milieu aqueux acide ayant un pH de 1,5 à 5, à raison de 1 à 10 parties en volume du milieu acide pour 1 partie en poids de fundus, à une température de 4 à 30°C et pendant une minute à 15 heures pour obtenir des matières solides et une solution aqueuse contenant une partie lipasique,
- à séparer la solution aqueuse des matières solides pour obtenir une solution aqueuse séparée,
- à ajouter à la solution aqueuse séparée une quantité suffisante de sel hydrosoluble et à l'y laisser pendant une durée suffisante pour relarguer l'extrait lipasique cherché et pour obtenir une solution surnageante, et
- à séparer l'extrait lipasique cherché de la solution surnageante et à le recueillir.
b) pour obtenir un extrait lipasique dessalé :
- à dissoudre l'extrait lipasique dans une phase aqueuse,
- à filtrer la phase aqueuse sur une membrane ayant un seuil de coupure compris entre 5000 et 10000 daltons pour obtenir une solution de l'extrait lipasique dessalé, et
- à lyophiliser la solution d'extrait lipasique dessalé en l'extrait lipasique dessalé.
c) pour obtenir un extrait lipasique délipidé
- à effectuer en outre sur l'une quelconque des parties solides contenant l'extrait lipasique que sont le fundus d'estomac, l'extrait lipasique et l'extrait lipasique dessalé une opération de délipidation.
d) pour obtenir un extrait lipasique enrichi non délipidé :
- à dissoudre l'extrait lipasique dans un tampon de pH compris entre 2 et 7, pour obtenir une solution tamponnée,
- à chromatographier la solution tamponnée de pH compris entre 2 et 7 sur un tamis moléculaire dont la limite d'exclusion est supérieure à 1.000.000 daltons et à recueillir la fraction d'élution exclue contenant l'extrait lipasique enrichi non délipidé en solution tamponnée,
- à filtrer cette fraction d'élution exclue sur une membrane ayant un seuil de coupure de 10.000 daltons pour obtenir une fraction dessalée, et
- à lyophiliser cette fraction dessalée en l'extrait lipasique enrichi non délipidé cherché.
e) pour obtenir un extrait lipasique enrichi, délipidé :
e1
- à dissoudre l'extrait lipasique dans de l'eau pour obtenir une solution aqueuse contenant l'extrait lipasique,
- à filtrer la solution aqueuse contenant l'extrait lipasique sur une membrane ayant un seuil de coupure compris entre 5000 et 10000 daltons pour obtenir une solution exempte de sel,
- à adsorber la solution exempte de sel sur un support échangeur d'ions,
- à désorber du support par un éluant dont on augmente la force ionique progressivement en fonction du temps,
- à recueillir une fraction d'élution ayant une activité lipasique et contenant l'extrait lipasique enrichi délipidé,
- à filtrer la fraction d'élution ayant une activité lipasique sur une membrane ayant un seuil de coupure compris entre 5000 et 10000 daltons pour obtenir une fraction d'élution dessalée, et
- à lyophiliser cette fraction d'élution dessalée en l'extrait lipasique enrichi délipidé cherché, ou
e2
- à chromatographier la solution tamponnée de pH compris entre 2 et 7 obtenue sous d ci-dessus sur un tamis moléculaire,
- à recueillir une fraction d'élution retenue correspondant à des masses moléculaires comprises entre 30000 et 55000 daltons et contenant l'extrait lipasique enrichi délipidé,
- à filtrer cette fraction d'élution retenue correspondant à des masses moléculaires comprises entre 30000 et 55000 et contenant l'extrait lipasique enrichi sur une membrane ayant un seuil de coupure compris entre 5000 et 10000 daltons pour obtenir une solution de l'extrait lipasique enrichi délipidé dessalé, et
- à lyophiliser cette solution de l'extrait lipasique enrichi délipidé dessalé en l'extrait lipasique enrichi délipidé cherché.
f) pour obtenir la lipase :
f1
- à partir de la fraction d'élution ayant une activité lipasique et contenant l'extrait lipasique enrichi délipidé obtenu en e1 ci-dessus,
- à la chromatographier sur un tamis moléculaire,
- à recueillir une fraction d'élution retenue correspondant à des masses moléculaires comprises entre 45000 et 55000 daltons et contenant la lipase,
- à filtrer cette fraction d'élution contenant la lipase sur une membrane ayant un seuil de coupure de 5000 à 10000 daltons pour obtenir une solution dessalée contenant la lipase, et
- à lyophiliser cette solution dessalée contenant la lipase, ou
f2
- à partir de la fraction d'élution retenue correspondant à des masses moléculaires comprises entre 30000 et 55000 daltons et contenant l'extrait lipasique enrichi délipidé obtenu en e2 ci-dessus,
- à l'adsorber sur un support échangeur d'ions,
- à désorber du support par un éluant dont on augmente la force ionique progressivement en fonction du temps,
- à recueillir une fraction d'élution ayant une activité lipasique et contenant la lipase,
- à filtrer la fraction d'élution ayant une activité lipasique et contenant la lipase sur une membrane ayant un seuil de coupure de 5000 à 10000 daltons pour obtenir une fraction d'élution dessalée contenant la lipase, et
- à lyophiliser cette fraction d'élution dessalée contenant la lipase en la lipase cherchée.

2. Procédé suivant la revendication 1, caractérisé en ce qu'il consiste à effectuer la mise en contact à une température comprise entre 0 et 20°C.

3. Procédé suivant la revendication 1 ou 2, caractérisé en ce qu'il consiste à séparer la solution aqueuse des matières solides par filtration, par décantation ou par centrifugation à une température inférieure à 25°C et, de préférence, à une température comprise entre 4 et 20°C.

4. Procédé suivant l'une des revendications 1 à 3, caractérisé en ce qu'il consiste à ajouter de 80 à 700 grammes de sels hydrosolubles et à les y laisser pendant 15 minutes à 20 heures à une température comprise entre 0 et 20°C.

5. Procédé suivant la revendication 4, caractérisé en ce que les sels sont des sels de magnésium, de potassium, de sodium et, de préférence, d'ammonium d'anions polyvalents, notamment de phosphate ou de sulfate.

6. Procédé suivant l'une des revendications précédentes, caractérisé en ce qu'il consiste à effectuer l'opération de délipidation en mettant les parties solides en contact avec des solvants aptes à solubiliser les matières grasses comme des hydrocarbures, des éthers, des cétones, des alcools ou des halogénures de carbone ayant un point d'ébullition inférieur à 80°C.

7. Procédé suivant l'une des revendications précédentes, caractérisé en ce qu'il consiste à effectuer les adsorptions sur support échangeur d'ions sur des supports échangeurs de cations.

8. Procédé suivant l'une des revendications précédentes, caractérisé en ce qu'il consiste à faire précéder la mise en contact avec le milieu aqueux acide d'une fragmentation du fundus à une dimension inférieure à 1 cm.

9. Une lipase ayant un poids moléculaire selon la technique de Laemmli de 49 000 daltons dont 9000 correspondent à des sucres et 40000 à une protéine, dont le nombre des types d'acides aminés est le suivant :
Asp. Asn : 45; Thr : 19; Ser : 28; Glx : 30; Pro : 29; Gly : 25; Ala : 28; Val : 27; Met : 8; Ile : 18; Leu : 26; Tyr : 16, Phe : 18 : Lys : 17; His : 7; Arg : 10; Cys : 9 et Trp : 6, dont la séquence N-terminale est :
Lys - Ser - Ala - Pro - Thr - Asn - Pro - Glu - Val - Asn - Met - X - Ile - Ser - Glu - Met - Ile - Ser - Tyr - Trp - Gly - Tyr - Pro - Ser - Glu - Lys - Tyr - Glu - Val - Val- X désignant un acide aminé non déterminé, dont l'activité lipasique spécifique selon la méthode de Gargouri est supérieure à 1000 U/mg de protéine, dont l'activité maximale est obtenue pour une valeur de pH de 4,5 environ, dont l'activité à des valeurs de pH de 3 et 7, est au moins égale à la moitié de l'activité maximale, dont l'activité se conserve après incubation pendant 2 heures, à 37°C et à une valeur de pH de 2,dont l'acide aminé impliqué dans l'activité lipolytique est la cystéine, qui résiste à la pepsine, est dégradée par la chymotrypsine et par la trypsine, dont le pH isoélectrique est entre 5,7 et 7,1 et qui est susceptible d'être préparée par le procédé suivant les revendications 1 à 8 à partir de fundus de l'estomac de lapin.

10. L'utilisation de la lipase et/ou d'un extrait lipasique tel que préparé à la revendication 1 ou tel que définie à la revendication 9 pour l'obtention d'un médicament destiné à lutter contre les malabsorptions des graisses chez l'homme et chez l'animal.

## Claims

1. Process for the preparation of lipase extracts or a lipase, characterized in that it comprises:
a) for obtaining a lipase extract:
- bringing the stomach fundus of an adult horse aged of at least three years or adult rabbit aged of at least two months into contact with an acid aqueous medium having a pH of 1.5 to 5, at a rate of 1 to 10 parts by volume of the acid medium for 1 part by weight of fundus, at a temperature of 4 to 30°C and for between 1 minute and 15 hours to obtain solid materials and an aqueous solution containing a lipase part,
- separating the aqueous solution from the solid materials to obtain a separate aqueous solution,
- adding to the separate aqueous solution an adequate quantity of hydrosoluble salt and leaving it there for a time adequate for salting out the sought lipase extract and for obtaining a supernatant solution and
- separating the sought lipase extract from the supernatant solution and collecting it;
b) for obtaining a desalted lipase extract:
- dissolving the lipase extract in an aqueous phase,
- filtering the aqueous phase on a membrane having a cutoff threshold between 5,000 and 10,000 daltons for obtaining a solution of the desalted lipase extract and
- lyophilizing the desalted lipase extract solution into desalted lipase extract;
c) for obtaining a delipidated lipase extract:
- also carrying out a delipidation operation on any random one of the solid parts containing the lipase extract constituted by the stomach fundus, the lipase extract and the desalted lipase extract;
d) for obtaining a non-delipidated, enriched lipase extract:
- dissolving the lipase extract in a pH buffer between 2 and 7 to obtain a buffered solution,
- subjecting the buffered solution with a pH between 2 and 7 to chromatography on a molecular sieve, whose exclusion limit exceeds 1,000,000 daltons and collecting the excluded elution fraction containing the non-delipidated, enriched lipase extract in buffered solution,
- filtering said excluded elution fraction on a membrane having a cutoff threshold of 10,000 daltons for obtaining a desalted fraction and
- lyophilizing this desalted fraction into the sought, non-delipidated, enriched lipase extract;
e) for obtaining a delipidated, enriched lipase extract:
- e)1 dissolving the lipase extract in water for obtaining an aqueous solution containing the lipase extract,
- filtering the aqueous solution containing the lipase extract on a membrane having a cutoff threshold between 5,000 and 10,000 daltons for obtaining a salt-free solution,
- adsorbing the salt-free solution on an ion exchange support,
- desorbing the support by an eluent, whose ionic strength is progressively increased as a function of time,
- collecting an elution fraction having a lipase activity and containing the delipidated, enriched lipase extract,
- filtering the elution fraction having a lipase activity on a membrane having a cutoff threshold between 5,000 and 10,000 daltons for obtaining a desalted elution fraction and
- lyophilizing said desalted elution fraction into the sought, delipidated, enriched lipase extract, or
e)2 Subjecting the buffered solution of pH between 2 and 7 obtained under d) hereinbefore to chromatography on a molecular sieve,
- collecting a retained elution fraction corresponding to molecular weights between 30,000 and 55,000 daltons and containing the delipidated, enriched lipase extract,
- filtering said retained elution fraction corresponding to molecular weights between 30,000 and 55,000 and containing the enriched lipase extract on a membrane having a cutoff threshold between 5,000 and 10,000 daltons for obtaining a desalted, delipidated, enriched lipase extract solution and
- lyophilizing said desalted, delipidated, enriched lipase extract solution into the sought, delipidated, enriched lipase extract;
f) for obtaining the lipase:
f)1 on the basis of the elution fraction having a lipase activity and containing the delipidated, enriched lipase extract obtained in e)1 hereinbefore,
- subjecting it to chromatography on a molecular sieve,
- collecting a retained elution fraction corresponding to molecular weights between 45,000 and 50,000 daltons and containing the lipase,
- filtering said elution fraction containing the lipase on a membrane having a cutoff threshold of 5,000 to 10,000 daltons for obtaining a desalted solution containing the lipase and
- lyophilizing said desalted solution containing the lipase, or
f)2 on the basis of the retained elution fraction corresponding to molecular weights between 30,000 and 55,000 daltons and containing the delipidated, enriched lipase extract obtained in e)2 hereinbefore,
- adsorbing it on an ion exchange support,
- desorbing the support by an eluent, whereof the ionic strength is progressively increased as a function of time,
- collecting an elution fraction having a lipase activity and containing the lipase,
- filtering the elution fraction having a lipase activity and containing the lipase on a membrane having a cutoff threshold of 5,000 to 10,000 daltons for obtaining a desalted elution fraction containing the lipase and
- lyophilizing said desalted elution fraction containing the lipase into the sought lipase.

2. Process according to claim 1, characterized in that it comprises of effecting contact at a temperature between 0 and 20°C.

3. Process according to claims 1 or 2, characterized in that it comprises separating the aqueous solution from the solid substances by filtration, decantation or centrifuging at a temperature below 25°C and preferably at between 4 and 20°C.

4. Process according to any one of the claims 1 to 3, characterized in that it comprises of adding 80 to 700 grams of hydrosoluble salts and leaving them for 15 minutes to 20 hours at a temperature between 0 and 20°C.

5. Process according to claim 4, characterized in that the salts are magnesium, potassium, sodium and preferably ammonium salts and polyvalent anions, particularly of phosphate or sulphate.

6. Process according to any one of the preceding claims, characterized in that it consists of performing the delipidation operation by bringing the solid parts into contact with solvents able to solubilize the fatty substances, such as hydrocarbons, ethers, ketones, alcohols or carbon halides having a boiling point below 80°C.

7. Process according to one of the preceding claims, characterized in that it comprises of performing adsorptions on an ion exchange support on cation exchange supports.

8. Process according to one of the preceding claims, characterized in that it comprises preceding the contacting with the acid aqueous medium by a fragmentation of the fundus to a size below 1 cm.

9. A lipase having a molecular weight according to the Laemmli method of 49,000 daltons, whereof 9,000 correspond to sugars and 40,000 to a protein, the number of amino acid types being as follows:
Asp. Asn : 45 : Thr : 19 : Ser : 28 : Glx : 30 : Pro : 29 : Gly : 25 : Ala : 28 : Val : 27 : Met : 8 : Ile : 18 : Leu : 26 : Tyr : 16 : Phe : 18 : Lys : 17 : His : 7 : Arg : 10 : Cys : 9 and Trp : 6, whereof the N-terminal sequence is:
Lys-Ser-Ala-Pro-Thr-Asn-Pro-Ser-Glü-Glu-Val-Asn-Met-X-Ile-Ser-Glu-Met-Ile-Ser-Tyr-Trp-Gly-Tyr-Pro-Lys-Tyr-Glu-Val-Val,
X designating an indeterminate amino acid, whose specific lipase activity according to the Gargouri method exceeds 1,000 U/mg of protein, whose maximum activity is obtained for a pH-value of approximately 4.5, whose activity, at pH-values of 3 and 7, is at least equal to half the maximum activity, whose activity is maintained after incubating for 2 hours, at 37°C and a pH-value of 2, whose amino acid involved in the lipolytic activity is cysteine, which is able to resist pepsin, is degraded by chymotrypsin and by trypsin, whose isoelectric pH is between 5.7 and 7.1 and which can be prepared by the process of claims 1 to 8 on the basis of rabbit stomach fundus.

10. Medicament for fighting against the malabsorption of fatty substances in man and animals, characterized in that it comprises, as the active principle, a lipase and/or a lipase extract according to claim 9.

## Claims (Claims for the following Contracting State(s): AT, ES)

1. Process for the preparation of lipase extracts or a lipase, characterized in that it comprises:
a) for obtaining a lipase extract:
- bringing the stomach fundus of an adult horse aged of at least three years or adult rabbit aged of at least two months into contact with an acid aqueous medium having a pH of 1.5 to 5, at a rate of 1 to 10 parts by volume of the acid medium for 1 part by weight of fundus, at a temperature of 4 to 30°C and for between 1 minute and 15 hours to obtain solid materials and an aqueous solution containing a lipase part,
- separating the aqueous solution from the solid materials to obtain a separate aqueous solution,
- adding to the separate aqueous solution an adequate quantity of hydrosoluble salt and leaving it there for a time adequate for salting out the sought lipase extract and for obtaining a supernatant solution and
- separating the sought lipase extract from the supernatant solution and collecting it;
b) for obtaining a desalted lipase extract:
- dissolving the lipase extract in an aqueous phase,
- filtering the aqueous phase on a membrane having a cutoff threshold between 5,000 and 10,000 daltons for obtaining a solution of the desalted lipase extract and
- lyophilizing the desalted lipase extract solution into desalted lipase extract;
c) for obtaining a delipidated lipase extract:
- also carrying out a delipidation operation on any random one of the solid parts containing the lipase extract constituted by the stomach fundus, the lipase extract and the desalted lipase extract;
d) for obtaining a non-delipidated, enriched lipase extract:
- dissolving the lipase extract in a pH buffer between 2 and 7 to obtain a buffered solution,
- subjecting the buffered solution with a pH between 2 and 7 to chromatography on a molecular sieve, whose exclusion limit exceeds 1,000,000 daltons and collecting the excluded elution fraction containing the non-delipidated, enriched lipase extract in buffered solution,
- filtering said excluded elution fraction on a membrane having a cutoff threshold of 10,000 daltons for obtaining a desalted fraction and
- lyophilizing this desalted fraction into the sought, non-delipidated, enriched lipase extract;
e) for obtaining a delipidated, enriched lipase extract:
- e)1 dissolving the lipase extract in water for obtaining an aqueous solution containing the lipase extract,
- filtering the aqueous solution containing the lipase extract on a membrane having a cutoff threshold between 5,000 and 10,000 daltons for obtaining a salt-free solution,
- adsorbing the salt-free solution on an ion exchange support,
- desorbing the support by an eluent, whose ionic strength is progressively increased as a function of time,
- collecting an elution fraction having a lipase activity and containing the delipidated, enriched lipase extract,
- filtering the elution fraction having a lipase activity on a membrane having a cutoff threshold between 5,000 and 10,000 daltons for obtaining a desalted elution fraction and
- lyophilizing said desalted elution fraction into the sought, delipidated, enriched lipase extract, or
e)2 Subjecting the buffered solution of pH between 2 and 7 obtained under d) hereinbefore to chromatography on a molecular sieve,
- collecting a retained elution fraction corresponding to molecular weights between 30,000 and 55,000 daltons and containing the delipidated, enriched lipase extract,
- filtering said retained elution fraction corresponding to molecular weights between 30,000 and 55,000 and containing the enriched lipase extract on a membrane having a cutoff threshold between 5,000 and 10,000 daltons for obtaining a desalted, delipidated, enriched lipase extract solution and
- lyophilizing said desalted, delipidated, enriched lipase extract solution into the sought, delipidated, enriched lipase extract;
f) for obtaining the lipase:
f)1 on the basis of the elution fraction having a lipase activity and containing the delipidated, enriched lipase extract obtained in e)1 hereinbefore,
- subjecting it to chromatography on a molecular sieve,
- collecting a retained elution fraction corresponding to molecular weights between 45,000 and 50,000 daltons and containing the lipase,
- filtering said elution fraction containing the lipase on a membrane having a cutoff threshold of 5,000 to 10,000 daltons for obtaining a desalted solution containing the lipase and
- lyophilizing said desalted solution containing the lipase, or
f)2 on the basis of the retained elution fraction corresponding to molecular weights between 30,000 and 55,000 daltons and containing the delipidated, enriched lipase extract obtained in e)2 hereinbefore,
- adsorbing it on an ion exchange support,
- desorbing the support by an eluent, whereof the ionic strength is progressively increased as a function of time,
- collecting an elution fraction having a lipase activity and containing the lipase,
- filtering the elution fraction having a lipase activity and containing the lipase on a membrane having a cutoff threshold of 5,000 to 10,000 daltons for obtaining a desalted elution fraction containing the lipase and
- lyophilizing said desalted elution fraction containing the lipase into the sought lipase.

2. Process according to claim 1, characterized in that it comprises of effecting contact at a temperature between 0 and 20°C.

3. Process according to claims 1 or 2, characterized in that it comprises separating the aqueous solution from the solid substances by filtration, decantation or centrifuging at a temperature below 25°C and preferably at between 4 and 20°C.

4. Process according to any one of the claims 1 to 3, characterized in that it comprises of adding 80 to 700 grams of hydrosoluble salts and leaving them for 15 minutes to 20 hours at a temperature between 0 and 20°C.

5. Process according to claim 4, characterized in that the salts are magnesium, potassium, sodium and preferably ammonium salts and polyvalent anions, particularly of phosphate or sulphate.

6. Process according to any one of the preceding claims, characterized in that it consists of performing the delipidation operation by bringing the solid parts into contact with solvents able to solubilize the fatty substances, such as hydrocarbons, ethers, ketones, alcohols or carbon halides having a boiling point below 80°C.

7. Process according to one of the preceding claims, characterized in that it comprises of performing adsorptions on an ion exchange support on cation exchange supports.

8. Process according to one of the preceding claims, characterized in that it comprises preceding the contacting with the acid aqueous medium by a fragmentation of the fundus to a size below 1 cm.

9. The use of the lipase and/or a lipase extract as prepared in claim 1 for preparing a medicament for fighting against the malabsorption of fatty substances in man and animals.

10. Process for preparing a medicament for fighting against the malabsorption of fatty substances in man and animals, characterized in that it comprises mixing the lipase and/or a lipase extract as prepared in claim 1 with a pharmaceutical carrier.

## Claims (Claims for the following Contracting State(s): GR)

1. Process for the preparation of lipase extracts or a lipase, characterized in that it comprises:
a) for obtaining a lipase extract:
- bringing the stomach fundus of an adult horse aged of at least three years or adult rabbit aged of at least two months into contact with an acid aqueous medium having a pH of 1.5 to 5, at a rate of 1 to 10 parts by volume of the acid medium for 1 part by weight of fundus, at a temperature of 4 to 30°C and for between 1 minute and 15 hours to obtain solid materials and an aqueous solution containing a lipase part,
- separating the aqueous solution from the solid materials to obtain a separate aqueous solution,
- adding to the separate aqueous solution an adequate quantity of hydrosoluble salt and leaving it there for a time adequate for salting out the sought lipase extract and for obtaining a supernatant solution and
- separating the sought lipase extract from the supernatant solution and collecting it;
b) for obtaining a desalted lipase extract:
- dissolving the lipase extract in an aqueous phase,
- filtering the aqueous phase on a membrane having a cutoff threshold between 5,000 and 10,000 daltons for obtaining a solution of the desalted lipase extract and
- lyophilizing the desalted lipase extract solution into desalted lipase extract;
c) for obtaining a delipidated lipase extract:
- also carrying out a delipidation operation on any random one of the solid parts containing the lipase extract constituted by the stomach fundus, the lipase extract and the desalted lipase extract;
d) for obtaining a non-delipidated, enriched lipase extract:
- dissolving the lipase extract in a pH buffer between 2 and 7 to obtain a buffered solution,
- subjecting the buffered solution with a pH between 2 and 7 to chromatography on a molecular sieve, whose exclusion limit exceeds 1,000,000 daltons and collecting the excluded elution fraction containing the non-delipidated, enriched lipase extract in buffered solution,
- filtering said excluded elution fraction on a membrane having a cutoff threshold of 10,000 daltons for obtaining a desalted fraction and
- lyophilizing this desalted fraction into the sought, non-delipidated, enriched lipase extract;
e) for obtaining a delipidated, enriched lipase extract:
- e)1 dissolving the lipase extract in water for obtaining an aqueous solution containing the lipase extract,
- filtering the aqueous solution containing the lipase extract on a membrane having a cutoff threshold between 5,000 and 10,000 daltons for obtaining a salt-free solution,
- adsorbing the salt-free solution on an ion exchange support,
- desorbing the support by an eluent, whose ionic strength is progressively increased as a function of time,
- collecting an elution fraction having a lipase activity and containing the delipidated, enriched lipase extract,
- filtering the elution fraction having a lipase activity on a membrane having a cutoff threshold between 5,000 and 10,000 daltons for obtaining a desalted elution fraction and
- lyophilizing said desalted elution fraction into the sought, delipidated, enriched lipase extract, or
e)2 Subjecting the buffered solution of pH between 2 and 7 obtained under d) hereinbefore to chromatography on a molecular sieve,
- collecting a retained elution fraction corresponding to molecular weights between 30,000 and 55,000 daltons and containing the delipidated, enriched lipase extract,
- filtering said retained elution fraction corresponding to molecular weights between 30,000 and 55,000 and containing the enriched lipase extract on a membrane having a cutoff threshold between 5,000 and 10,000 daltons for obtaining a desalted, delipidated, enriched lipase extract solution and
- lyophilizing said desalted, delipidated, enriched lipase extract solution into the sought, delipidated, enriched lipase extract;
f) for obtaining the lipase:
f)1 on the basis of the elution fraction having a lipase activity and containing the delipidated, enriched lipase extract obtained in e)1 hereinbefore,
- subjecting it to chromatography on a molecular sieve,
- collecting a retained elution fraction corresponding to molecular weights between 45,000 and 50,000 daltons and containing the lipase,
- filtering said elution fraction containing the lipase on a membrane having a cutoff threshold of 5,000 to 10,000 daltons for obtaining a desalted solution containing the lipase and
- lyophilizing said desalted solution containing the lipase, or
f)2 on the basis of the retained elution fraction corresponding to molecular weights between 30,000 and 55,000 daltons and containing the delipidated, enriched lipase extract obtained in e)2 hereinbefore,
- adsorbing it on an ion exchange support,
- desorbing the support by an eluent, whereof the ionic strength is progressively increased as a function of time,
- collecting an elution fraction having a lipase activity and containing the lipase,
- filtering the elution fraction having a lipase activity and containing the lipase on a membrane having a cutoff threshold of 5,000 to 10,000 daltons for obtaining a desalted elution fraction containing the lipase and
- lyophilizing said desalted elution fraction containing the lipase into the sought lipase.

2. Process according to claim 1, characterized in that it comprises of effecting contact at a temperature between 0 and 20°C.

3. Process according to claims 1 or 2, characterized in that it comprises separating the aqueous solution from the solid substances by filtration, decantation or centrifuging at a temperature below 25°C and preferably at between 4 and 20°C.

4. Process according to any one of the claims 1 to 3, characterized in that it comprises of adding 80 to 700 grams of hydrosoluble salts and leaving them for 15 minutes to 20 hours at a temperature between 0 and 20°C.

5. Process according to claim 4, characterized In that the salts are magnesium, potassium, sodium and preferably ammonium salts and polyvalent anions, particularly of phosphate or sulphate.

6. Process according to any one of the preceding claims, characterized in that it consists of performing the delipidation operation by bringing the solid parts into contact with solvents able to solubilize the fatty substances, such as hydrocarbons, ethers, ketones, alcohols or carbon halides having a boiling point below 80°C.

7. Process according to one of the preceding claims, characterized in that it comprises of performing adsorptions on an ion exchange support on cation exchange supports.

8. Process according to one of the preceding claims, characterized in that it comprises preceding the contacting with the acid aqueous medium by a fragmentation of the fundus to a size below 1 cm.

9. A lipase having a molecular weight according to the Laemmli method of 49,000 daltons, whereof 9,000 correspond to sugars and 40,000 to a protein, the number of amino acid types being as follows:
Asp. Asn : 45 : Thr : 19 : Ser : 28 : Glx : 30 : Pro : 29 : Gly : 25 : Ala : 28 : Val : 27 : Met : 8 : Ile : 18 : Leu : 26 : Tyr : 16 : Phe : 18 : Lys : 17 : His : 7 : Arg : 10 : Cys : 9 and Trp : 6, whereof the N-terminal sequence is:
Lys-Ser-Ala-Pro-Thr-Asn-Pro-Ser-Glü-Glu-Val-Asn-Met-X-Ile-Ser-Glu-Met-Ile-Ser-Tyr-Trp-Gly-Tyr-Pro-Lys-Tyr-Glu-Val-Val,
X designating an indeterminate amino acid, whose specific lipase activity according to the Gargouri method exceeds 1,000 U/mg of protein, whose maximum activity is obtained for a pH-value of approximately 4.5, whose activity, at pH-values of 3 and 7, is at least equal to half the maximum activity, whose activity is maintained after incubating for 2 hours, at 37°C and a pH-value of 2, whose amino acid involved in the lipolytic activity is cysteine, which is able to resist pepsin, is degraded by chymotrypsin and by trypsin, whose isoelectric pH is between 5.7 and 7.1 and which can be prepared by the process of claims 1 to 8 on the basis of rabbit stomach fundus.

10. The use of the lipase and/or a lipase extract as prepared in claim 1 or as defined in claim 9 for preparing a medicament for fighting against the malabsorption of fatty substances in man and animals.

## Patentansprüche

1. Verfahren zur Herstellung von Lipaseextrakten oder einer Lipase,
gekennzeichnet durch die Verfahrensschritte:
a) Erzeugung eines Lipaseextraktes:
- der Magenfundus von einem erwachsenen, mindesten 2 Monate alten Kaninchen oder von einem erwachsenen, mindestens 3 Jahre alten Pferd wird mit einem wäßrigen, sauren Medium mit einem pH-Wert von 1,5 bis 5 in einem Verhältnis 1 bis 10 Vol.-Teile saures Medium auf 1 Gew.-Teil Fundus 1 Minute bis 15 Stunden lang bei einer Temperatur von 4 bis 30° C behandelt, um Feststoffe und eine wäßrige Lösung zu erhalten, die einen Lipaseanteil aufweist;
- die wäßrige Lösung wird von den Feststoffen abgetrennt, um eine abgetrennte wäßrige Lösung zu erhalten;
- zu der abgetrennten wäßrigen Lösung wird eine ausreichende Menge wasserlösliches Salz hinzugefügt und ausreichend lange in der Lösung belassen, um den gesuchten Lipaseextrakt auszusalzen und um einen Überstand zu erzeugen; und
- der gesuchte Lipaseextrakt wird von dem Überstand abgetrennt und gesammelt;
b) Erzeugung eines entsalzten Lipaseextraktes:
- der Lipaseextrakt wird in einer wäßrigen Phase gelöst;
- die wäßrige Phase wird über eine Membran filtriert, die eine Durchlaufschwelle für Molmassen von 5000 bis 10 000 Dalton bildet, um eine Lösung des entsalzten Lipaseextraktes zu erhalten; und
- die Lösung des entsalzten Lipaseextraktes wird lyophilisiert, um einen entsalzten Lipaseextrakt zu erhalten;
c) Erzeugung eines entfetteten Lipaseextraktes:
- irgendein Lipaseextrakt-haltiger fester Anteil, Magenfundus, Lipaseextrakt und entsalzter Lipaseextrakt, werden entfettet;
d) Erzeugung eines angereicherten, nicht entfetteten Lipaseextraktes:
- der Lipaseextrakt wird in einer Pufferlösung mit einem pH-Wert von 2 und bis 7 aufgelöst, um eine gepufferte Lipaseextrakt-Lösung zu erhalten;
- die gepufferte Lösung wird über ein Molekularsieb chromatographiert, das Molmassen oberhalb 1 000 000 Dalton abtrennt, und die nicht festgehaltene Elutionsfraktion wird gesammelt, die den angereicherten, nicht entfetteten Lipaseextrakt in gepufferter Lösung enthält;
- diese nicht festgehaltene Elutionsfraktion wird über eine Membran filtriert, die eine Durchlaufschwelle für Molmassen von 5000 bis 10 000 Dalton bildet, um eine entsalzte Fraktion zu erhalten; und
- diese entsalzte Fraktion wird lyophilisiert, um den gesuchten, angereicherten, nicht entfetteten Lipaseextrakt zu erhalten;
e) Erzeugung eines angereicherten, entfetteten Lipaseextraktes:
e1)
- der Lipaseextrakt wird in Wasser aufgelöst, um eine wäßrige, Lipaseextrakt-haltige Lösung zu erhalten;
- die Lipaseextrakt-haltige, wäßrige Lösung wird über eine Membran filtriert, die eine Durchlaufschwelle für Molmassen von 5000 und bis 10 000 Dalton bildet, um eine salzfreie Lösung zu erhalten;
- die salzfreie Lösung wird an einem Matrix-Ionenaustauscher adsorbiert;
- von dem Matrix-Ionenaustauscher wird mit einem Laufmittel desorbiert, dessen Ionenstärke schrittweise in Abhängigkeit von der Zeit erhöht wird;
- diejenige Elutionsfraktion wird gesammelt, die eine Lipaseaktivität aufweist und die den angereicherten, entfetteten Lipaseextrakt enthält;
- diese Lipaseaktivität aufweisende Elutionsfraktion wird über eine Membran filtriert, die eine Durchlaufschwelle für Molmassen von 5000 bis 10 000 Dalton bildet, um eine entsalzte Elutionsfraktion zu erhalten; und
- diese entsalzte Elutionsfraktion wird lyophilisiert, um den gesuchten, angereicherten, entfetteten Lipaseextrakt zu erhalten; oder
e2)
- die oben unter (d) erhaltene gepufferte Lösung mit einem pH-Wert von 2 bis 7 wird über ein Molekularsieb chromatographiert;
- es wird die festgehaltene Elutionsfraktion gesammelt, die Molmassen zwischen 30 000 und 55 000 Dalton entspricht und die den angereicherten, entfetteten Lipaseextrakt enthält;
- diese festgehaltene Elutionsfraktion wird über eine Membran filtriert, die eine Durchlaufschwelle für Molmassen von 5 000 bis 10 000 Dalton bildet, um eine Lösung des angereicherten, entfetteten, entsalzten Lipaseextraktes zu erhalten; und
- diese Lipaseextrakt-Lösung wird lyophilisiert, um den gesuchten, angereicherten, entfetteten Lipaseextrakt zu erhalten;
f) zur Erzeugung der Lipase:
f1)
- es wird von der oben unter (e1) erhaltenen Elutionsfraktion ausgegangen, die Lipaseaktivität aufweist und die den angereicherten, entfetteten Lipaseextrakt enthält;
- diese Elutionsfraktion wird über ein Molekularsieb chromatographiert;
- eine festgehaltene Elutionsfraktion wird gesammelt, die den Molmassen von 45 000 bis 55 000 Dalton entspricht und die die Lipase enthält;
- diese Lipase-haltige Elutionsfraktion wird über eine Membran filtriert, die eine Durchlaufschwelle für Molmassen von 5000 bis 10 000 Dalton darstellt, um eine entsalzte, Lipase-haltige Lösung zu erhalten; und
- diese entsalzte, Lipase-haltige Lösung wird lyophilisiert; oder
f2)
- es wird von der oben unter (e2) erhaltenen Elutionsfraktion ausgegangen, die den Molmassen von 30 000 bis 55 000 Dalton entspricht und die den angereicherten, entfetteten Lipaseextrakt enthält;
- diese Elutionsfraktion wird an einen Matrix-Ionenaustauscher adsorbiert;
- von dem Ionentauscher wird mit einem Laufmittel desorbiert, dessen Ionenstärke schrittweise in Abhängigkeit von der Zeit erhöht wird;
- diejenige Elutionsfraktion wird gesammelt, die Lipaseaktivität aufweist und die die Lipase enthält;
- die die Lipaseaktivität aufweisende Elutionsfraktion wird über eine Membran filtriert, die eine Durchlaufschwelle für Molmassen von 5000 bis 10 000 Dalton bildet, um eine entsalzte, Lipase-haltige Elutionsfraktion zu erhalten; und
- diese entsalzte, Lipase-haltige Elutionsfraktion wird lyophilisiert, um die gesuchte Lipase zu erhalten.

2. Verfahren nach Anspruch 1,
dadurch gekennzeichnet, daß
die Behandlung bei einer Temperatur zwischen 0 und 20° C durchgeführt wird.

3. Verfahren nach Anspruch 1 oder 2,
dadurch gekennzeichnet, daß
die wäßrige Lösung von den Feststoffen durch Filtrieren, Dekantieren oder Zentrifugieren abgetrennt wird, bei einer Temperatur unterhalb von 25° C, vorzugsweise bei einer Temperatur zwischen 4 und 20° C.

4. Verfahren nach einem der Ansprüche 1 bis 3,
dadurch gekennzeichnet, daß
80 bis 700 g wasserlösliche Salze zugesetzt werden und 15 Minuten bis 20 Stunden lang bei einer Temperatur zwischen 0 und 20° C in der Lösung belassen werden.

5. Verfahren nach Anspruch 4,
dadurch gekennzeichnet, daß
als Salze Magnesium-, Kalium-, Natrium- und vorzugsweise Ammoniumsalze mit mehrwertigen Anionen, insbesondere deren Phosphate oder Sulfate, eingesetzt werden.

6. Verfahren nach einem der Ansprüche 1 bis 5,
dadurch gekennzeichnet, daß
zur Entfettung die festen Bestandteile mit Lösungsmitteln behandelt werden, die zur Solubilisierung der Fette geeignet sind, wie Kohlenwasserstoffe, Ether, Ketone, Alkohole oder halogenierte Kohlenwasserstoffe, die einen Siedepunkt unterhalb 80° C aufweisen.

7. Verfahren nach einem der Ansprüche 1 bis 6,
dadurch gekennzeichnet, daß
die Adsorptionen an einem Matrix-Ionenaustauscher an einem Matrix-Kationenaustauscher durchgeführt werden.

8. Verfahren nach einem der Ansprüche 1 bis 7,
dadurch gekennzeichnet, daß
der Fundus vor der Behandlung mit dem wäßrigen, sauren Medium zerkleinert wird zu Teilchen mit einer Teilchengröße kleiner 1 cm.

9. Lipase,
mit einem Molekulargewicht von 49 000 Dalton, gemäß dem Verfahren von Laemmli, wovon 9000 Molmassen auf Zucker und 40 000 Molmassen auf ein Protein entfallen,
das nachstehende Anzahl der Aminosäure-Typen aufweist:
Asp. Asn: 45; Thr: 19; Ser: 28; Glx: 30; Pro: 29; Gly: 25; Ala: 28; Val: 27; Met: 8; Ile: 18; Leu: 26; Tyr: 16; Phe: 18; Lys: 17; His: 7; Arg: 10; Cys: 9 und Trp: 6;
das nachstehende N-terminale Sequenz aufweist:
Lys - Ser - Ala - Pro - Thr - Asn - Pro - Glu - Val - Asn -Met - X - Ile - Ser - Glu - Met - Ile - Ser - Tyr - Trp - Gly - Tyr - Pro - Ser - Glu - Lys - Tyr - Glu - Val -Val-, wobei X eine nicht bestimmte Aminosäure bezeichnet;
das eine spezifische Lipaseaktivität nach dem Verfahren von Gargouri über 1000 U/mg Protein aufweist;
das eine maximale Aktivität bei einem pH-Wert von etwa 4,5 aufweist;
dessen Aktivität bei pH-Werten von 3 bis 7 mindestens gleich der Hälfte der maximalen Aktivität ist;
dessen Aktivität nach einer 2 Stunden langen Inkubation bei 37° C und pH 2 erhalten bleibt;
bei dem die an der lipolytischen Aktivität beteiligte Aminosäure Cystein ist;
das gegen Pepsin resistent ist und das von Chymotrypsin sowie Trypsin abgebaut wird;
dessen isoelektrischer pH-Wert zwischen 5,7 und 7,1 liegt;und
das, ausgehend vom Magenfundus eines Kaninchens, leicht durch ein Verfahren nach einem der Ansprüche 1 bis 8 erhältlich ist.

10. Medikament gegen mangelhafte Absorptionen von Fetten bei Mensch und Tier,
dadurch gekennzeichnet, daß
das Medikament als Wirkstoff eine Lipase und/oder einen Lipaseextrakt nach Anspruch 9 enthält.

## Patentansprüche (Patentansprüche für folgende(n) Vertragsstaat(en): AT, ES)

1. Verfahren zur Herstellung von Lipaseextrakten oder einer Lipase,
gekennzeichnet durch die Verfahrensschritte:
a) Erzeugung eines Lipaseextraktes:
- der Magenfundus von einem erwachsenen, mindesten 2 Monate alten Kaninchen oder von einem erwachsenen, mindestens 3 Jahre alten Pferd wird mit einem wäßrigen, sauren Medium mit einem pH-Wert von 1,5 bis 5 in einem Verhältnis 1 bis 10 Vol.-Teile saures Medium auf 1 Gew.-Teil Fundus 1 Minute bis 15 Stunden lang bei einer Temperatur von 4 bis 30° C behandelt, um Feststoffe und eine wäßrige Lösung zu erhalten, die einen Lipaseanteil aufweist;
- die wäßrige Lösung wird von den Feststoffen abgetrennt, um eine abgetrennte wäßrige Lösung zu erhalten;
- zu der abgetrennten wäßrigen Lösung wird eine ausreichende Menge wasserlösliches Salz hinzugefügt und ausreichend lange in der Lösung belassen, um den gesuchten Lipaseextrakt auszusalzen und um einen Überstand zu erzeugen; und
- der gesuchte Lipaseextrakt wird von dem Überstand abgetrennt und gesammelt;
b) Erzeugung eines entsalzten Lipaseextraktes:
- der Lipaseextrakt wird in einer wäßrigen Phase gelöst;
- die wäßrige Phase wird über eine Membran filtriert, die eine Durchlaufschwelle für Molmassen von 5000 bis 10 000 Dalton bildet, um eine Lösung des entsalzten Lipaseextraktes zu erhalten; und
- die Lösung des entsalzten Lipaseextraktes wird lyophilisiert, um einen entsalzten Lipaseextrakt zu erhalten;
c) Erzeugung eines entfetteten Lipaseextraktes:
- irgendein Lipaseextrakt-haltiger fester Anteil, Magenfundus, Lipaseextrakt und entsalzter Lipaseextrakt, werden entfettet;
d) Erzeugung eines angereicherten, nicht entfetteten Lipaseextraktes:
- der Lipaseextrakt wird in einer Pufferlösung mit einem pH-Wert von 2 und bis 7 aufgelöst, um eine gepufferte Lipaseextrakt-Lösung zu erhalten;
- die gepufferte Lösung wird über ein Molekularsieb chromatographiert, das Molmassen oberhalb 1 000 000 Dalton abtrennt, und die nicht festgehaltene Elutionsfraktion wird gesammelt, die den angereicherten, nicht entfetteten Lipaseextrakt in gepufferter Lösung enthält;
- diese nicht festgehaltene Elutionsfraktion wird über eine Membran filtriert, die eine Durchlaufschwelle für Molmassen von 5000 bis 10 000 Dalton bildet, um eine entsalzte Fraktion zu erhalten; und
- diese entsalzte Fraktion wird lyophilisiert, um den gesuchten, angereicherten, nicht entfetteten Lipaseextrakt zu erhalten;
e) Erzeugung eines angereicherten, entfetteten Lipaseextraktes:
e1)
- der Lipaseextrakt wird in Wasser aufgelöst, um eine wäßrige, Lipaseextrakt-haltige Lösung zu erhalten;
- die Lipaseextrakt-haltige, wäßrige Lösung wird über eine Membran filtriert, die eine Durchlaufschwelle für Molmassen von 5000 und bis 10 000 Dalton bildet, um eine salzfreie Lösung zu erhalten;
- die salzfreie Lösung wird an einem Matrix-Ionenaustauscher adsorbiert;
- von dem Matrix-Ionenaustauscher wird mit einem Laufmittel desorbiert, dessen Ionenstärke schrittweise in Abhängigkeit von der Zeit erhöht wird;
- diejenige Elutionsfraktion wird gesammelt, die eine Lipaseaktivität aufweist und die den angereicherten, entfetteten Lipaseextrakt enthält;
- diese Lipaseaktivität aufweisende Elutionsfraktion wird über eine Membran filtriert, die eine Durchlaufschwelle für Molmassen von 5000 bis 10 000 Dalton bildet, um eine entsalzte Elutionsfraktion zu erhalten; und
- diese entsalzte Elutionsfraktion wird lyophilisiert, um den gesuchten, angereicherten, entfetteten Lipaseextrakt zu erhalten; oder
e2)
- die oben unter (d) erhaltene gepufferte Lösung mit einem pH-Wert von 2 bis 7 wird über ein Molekularsieb chromatographiert;
- es wird die festgehaltene Elutionsfraktion gesammelt, die Molmassen zwischen 30 000 und 55 000 Dalton entspricht und die den angereicherten, entfetteten Lipaseextrakt enthält;
- diese festgehaltene Elutionsfraktion wird über eine Membran filtriert, die eine Durchlaufschwelle für Molmassen von 5 000 bis 10 000 Dalton bildet, um eine Lösung des angereicherten, entfetteten, entsalzten Lipaseextraktes zu erhalten; und
- diese Lipaseextrakt-Lösung wird lyophilisiert, um den gesuchten, angereicherten, entfetteten Lipaseextrakt zu erhalten;
f) zur Erzeugung der Lipase:
f1)
- es wird von der oben unter (e1) erhaltenen Elutionsfraktion ausgegangen, die Lipaseaktivität aufweist und die den angereicherten, entfetteten Lipaseextrakt enthält;
- diese Elutionsfraktion wird über ein Molekularsieb chromatographiert;
- eine festgehaltene Elutionsfraktion wird gesammelt, die den Molmassen von 45 000 bis 55 000 Dalton entspricht und die die Lipase enthält;
- diese Lipase-haltige Elutionsfraktion wird über eine Membran filtriert, die eine Durchlaufschwelle für Molmassen von 5000 bis 10 000 Dalton darstellt, um eine entsalzte, Lipase-haltige Lösung zu erhalten; und
- diese entsalzte, Lipase-haltige Lösung wird lyophilisiert; oder
f2)
- es wird von der oben unter (e2) erhaltenen Elutionsfraktion ausgegangen, die den Molmassen von 30 000 bis 55 000 Dalton entspricht und die den angereicherten, entfetteten Lipaseextrakt enthält;
- diese Elutionsfraktion wird an einen Matrix-Ionenaustauscher adsorbiert;
- von dem Ionentauscher wird mit einem Laufmittel desorbiert, dessen Ionenstärke schrittweise in Abhängigkeit von der Zeit erhöht wird;
- diejenige Elutionsfraktion wird gesammelt, die Lipaseaktivität aufweist und die die Lipase enthält;
- die die Lipaseaktivität aufweisende Elutionsfraktion wird über eine Membran filtriert, die eine Durchlaufschwelle für Molmassen von 5000 bis 10 000 Dalton bildet, um eine entsalzte, Lipase-haltige Elutionsfraktion zu erhalten; und
- diese entsalzte, Lipase-haltige Elutionsfraktion wird lyophilisiert, um die gesuchte Lipase zu erhalten.

2. Verfahren nach Anspruch 1,
dadurch gekennzeichnet, daß
die Behandlung bei einer Temperatur zwischen 0 und 20° C durchgeführt wird.

3. Verfahren nach Anspruch 1 oder 2,
dadurch gekennzeichnet, daß
die wäßrige Lösung von den Feststoffen durch Filtrieren, Dekantieren oder Zentrifugieren abgetrennt wird, bei einer Temperatur unterhalb von 25° C, vorzugsweise bei einer Temperatur zwischen 4 und 20° C.

4. Verfahren nach einem der Ansprüche 1 bis 3,
dadurch gekennzeichnet, daß
80 bis 700 g wasserlösliche Salze zugesetzt werden und 15 Minuten bis 20 Stunden lang bei einer Temperatur zwischen 0 und 20° C in der Lösung belassen werden.

5. Verfahren nach Anspruch 4,
dadurch gekennzeichnet, daß
als Salze Magnesium-, Kalium-, Natrium- und vorzugsweise Ammoniumsalze mit mehrwertigen Anionen, insbesondere deren Phosphate oder Sulfate, eingesetzt werden.

6. Verfahren nach einem der Ansprüche 1 bis 5,
dadurch gekennzeichnet, daß
zur Entfettung die festen Bestandteile mit Lösungsmitteln behandelt werden, die zur Solubilisierung der Fette geeignet sind, wie Kohlenwasserstoffe, Ether, Ketone, Alkohole oder halogenierte Kohlenwasserstoffe, die einen Siedepunkt unterhalb 80° C aufweisen.

7. Verfahren nach einem der Ansprüche 1 bis 6,
dadurch gekennzeichnet, daß
die Adsorptionen an einem Matrix-Ionenaustauscher an einem Matrix-Kationenaustauscher durchgeführt werden.

8. Verfahren nach einem der Ansprüche 1 bis 7,
dadurch gekennzeichnet, daß
der Fundus vor der Behandlung mit dem wäßrigen, sauren Medium zerkleinert wird zu Teilchen mit einer Teilchengröße kleiner 1 cm.

9. Verwendung der Lipase und/oder eines Lipaseextraktes, die nach Anspruch 1 hergestellt sind, zur Erzeugung eines Medikamentes gegen mangelhafte Absorptionen von Fetten bei Mensch und Tier.

10. Verfahren zur Herstellung eines Medikaments gegen mangelhafte Absorptionen von Fetten bei Mensch und Tier
dadurch gekennzeichnet, daß
die/der nach Anspruch 1 hergestellte Lipase und/oder Lipaseextrakt mit einem pharmazeutischen Tägerstoff vermischt werden.

## Patentansprüche (Patentansprüche für folgende(n) Vertragsstaat(en): GR)

1. Verfahren zur Herstellung von Lipaseextrakten oder einer Lipase,
gekennzeichnet durch die Verfahrensschritte:
a) Erzeugung eines Lipaseextraktes:
- der Magenfundus von einem erwachsenen, mindesten 2 Monate alten Kaninchen oder von einem erwachsenen, mindestens 3 Jahre alten Pferd wird mit einem wäßrigen, sauren Medium mit einem pH-Wert von 1,5 bis 5 in einem Verhältnis 1 bis 10 Vol.-Teile saures Medium auf 1 Gew.-Teil Fundus 1 Minute bis 15 Stunden lang bei einer Temperatur von 4 bis 30° C behandelt, um Feststoffe und eine wäßrige Lösung zu erhalten, die einen Lipaseanteil aufweist;
- die wäßrige Lösung wird von den Feststoffen abgetrennt, um eine abgetrennte wäßrige Lösung zu erhalten;
- zu der abgetrennten wäßrigen Lösung wird eine ausreichende Menge wasserlösliches Salz hinzugefügt und ausreichend lange in der Lösung belassen, um den gesuchten Lipaseextrakt auszusalzen und um einen Überstand zu erzeugen; und
- der gesuchte Lipaseextrakt wird von dem Überstand abgetrennt und gesammelt;
b) Erzeugung eines entsalzten Lipaseextraktes:
- der Lipaseextrakt wird in einer wäßrigen Phase gelöst;
- die wäßrige Phase wird über eine Membran filtriert, die eine Durchlaufschwelle für Molmassen von 5000 bis 10 000 Dalton bildet, um eine Lösung des entsalzten Lipaseextraktes zu erhalten; und
- die Lösung des entsalzten Lipaseextraktes wird lyophilisiert, um einen entsalzten Lipaseextrakt zu erhalten;
c) Erzeugung eines entfetteten Lipaseextraktes:
- irgendein Lipaseextrakt-haltiger fester Anteil, Magenfundus, Lipaseextrakt und entsalzter Lipaseextrakt, werden entfettet;
d) Erzeugung eines angereicherten, nicht entfetteten Lipaseextraktes:
- der Lipaseextrakt wird in einer Pufferlösung mit einem pH-Wert von 2 und bis 7 aufgelöst, um eine gepufferte Lipaseextrakt-Lösung zu erhalten;
- die gepufferte Lösung wird über ein Molekularsieb chromatographiert, das Molmassen oberhalb 1 000 000 Dalton abtrennt, und die nicht festgehaltene Elutionsfraktion wird gesammelt, die den angereicherten, nicht entfetteten Lipaseextrakt in gepufferter Lösung enthält;
- diese nicht festgehaltene Elutionsfraktion wird über eine Membran filtriert, die eine Durchlaufschwelle für Molmassen von 5000 bis 10 000 Dalton bildet, um eine entsalzte Fraktion zu erhalten; und
- diese entsalzte Fraktion wird lyophilisiert, um den gesuchten, angereicherten, nicht entfetteten Lipaseextrakt zu erhalten;
e) Erzeugung eines angereicherten, entfetteten Lipaseextraktes:
e1)
- der Lipaseextrakt wird in Wasser aufgelöst, um eine wäßrige, Lipaseextrakt-haltige Lösung zu erhalten;
- die Lipaseextrakt-haltige, wäßrige Lösung wird über eine Membran filtriert, die eine Durchlaufschwelle für Molmassen von 5000 und bis 10 000 Dalton bildet, um eine salzfreie Lösung zu erhalten;
- die salzfreie Lösung wird an einem Matrix-Ionenaustauscher adsorbiert;
- von dem Matrix-Ionenaustauscher wird mit einem Laufmittel desorbiert, dessen Ionenstärke schrittweise in Abhängigkeit von der Zeit erhöht wird;
- diejenige Elutionsfraktion wird gesammelt, die eine Lipaseaktivität aufweist und die den angereicherten, entfetteten Lipaseextrakt enthält;
- diese Lipaseaktivität aufweisende Elutionsfraktion wird über eine Membran filtriert, die eine Durchlaufschwelle für Molmassen von 5000 bis 10 000 Dalton bildet, um eine entsalzte Elutionsfraktion zu erhalten; und
- diese entsalzte Elutionsfraktion wird lyophilisiert, um den gesuchten, angereicherten, entfetteten Lipaseextrakt zu erhalten; oder
e2)
- die oben unter (d) erhaltene gepufferte Lösung mit einem pH-Wert von 2 bis 7 wird über ein Molekularsieb chromatographiert;
- es wird die festgehaltene Elutionsfraktion gesammelt, die Molmassen zwischen 30 000 und 55 000 Dalton entspricht und die den angereicherten, entfetteten Lipaseextrakt enthält;
- diese festgehaltene Elutionsfraktion wird über eine Membran filtriert, die eine Durchlaufschwelle für Molmassen von 5 000 bis 10 000 Dalton bildet, um eine Lösung des angereicherten, entfetteten, entsalzten Lipaseextraktes zu erhalten; und
- diese Lipaseextrakt-Lösung wird lyophilisiert, um den gesuchten, angereicherten, entfetteten Lipaseextrakt zu erhalten;
f) zur Erzeugung der Lipase:
f1)
- es wird von der oben unter (e1) erhaltenen Elutionsfraktion ausgegangen, die Lipaseaktivität aufweist und die den angereicherten, entfetteten Lipaseextrakt enthält;
- diese Elutionsfraktion wird über ein Molekularsieb chromatographiert;
- eine festgehaltene Elutionsfraktion wird gesammelt, die den Molmassen von 45 000 bis 55 000 Dalton entspricht und die die Lipase enthält;
- diese Lipase-haltige Elutionsfraktion wird über eine Membran filtriert, die eine Durchlaufschwelle für Molmassen von 5000 bis 10 000 Dalton bildet, um eine entsalzte, Lipase-haltige Lösung zu erhalten; und
- diese entsalzte, Lipase-haltige Lösung wird lyophilisiert; oder
f2)
- es wird von der oben unter (e2) erhaltenen Elutionsfraktion ausgegangen, die den Molmassen von 30 000 bis 55 000 Dalton entspricht und die den angereicherten, entfetteten Lipaseextrakt enthält;
- diese Elutionsfraktion wird an einen Matrix-Ionenaustauscher adsorbiert;
- von dem Ionentauscher wird mit einem Laufmittel desorbiert, dessen Ionenstärke schrittweise in Abhängigkeit von der Zeit erhöht wird;
- diejenige Elutionsfraktion wird gesammelt, die Lipaseaktivität aufweist und die die Lipase enthält;
- die die Lipaseaktivität aufweisende Elutionsfraktion wird über eine Membran filtriert, die eine Durchlaufschwelle für Molmassen von 5000 bis 10 000 Dalton bildet, um eine entsalzte, Lipase-haltige Elutionsfraktion zu erhalten; und
- diese entsalzte, Lipase-haltige Elutionsfraktion wird lyophilisiert, um die gesuchte Lipase zu erhalten.

2. Verfahren nach Anspruch 1,
dadurch gekennzeichnet, daß
die Behandlung bei einer Temperatur zwischen 0 und 20° C durchgeführt wird.

3. Verfahren nach Anspruch 1 oder 2,
dadurch gekennzeichnet, daß
die wäßrige Lösung von den Feststoffen durch Filtrieren, Dekantieren oder Zentrifugieren abgetrennt wird, bei einer Temperatur unterhalb von 25° C, vorzugsweise bei einer Temperatur zwischen 4 und 20° C.

4. Verfahren nach einem der Ansprüche 1 bis 3,
dadurch gekennzeichnet, daß
80 bis 700 g wasserlösliche Salze zugesetzt werden und 15 Minuten bis 20 Stunden lang bei einer Temperatur zwischen 0 und 20° C in der Lösung belassen werden.

5. Verfahren nach Anspruch 4,
dadurch gekennzeichnet, daß
als Salze Magnesium-, Kalium-, Natrium- und vorzugsweise Ammoniumsalze mit mehrwertigen Anionen, insbesondere deren Phosphate oder Sulfate, eingesetzt werden.

6. Verfahren nach einem der Ansprüche 1 bis 5,
dadurch gekennzeichnet, daß
zur Entfettung die festen Bestandteile mit Lösungsmitteln behandelt werden, die zur Solubilisierung der Fette geeignet sind, wie Kohlenwasserstoffe, Ether, Ketone, Alkohole oder halogenierte Kohlenwasserstoffe, die einen Siedepunkt unterhalb 80° C aufweisen.

7. Verfahren nach einem der Ansprüche 1 bis 6,
dadurch gekennzeichnet, daß
die Adsorptionen an einem Matrix-Ionenaustauscher an einem Matrix-Kationenaustauscher durchgeführt werden.

8. Verfahren nach einem der Ansprüche 1 bis 7,
dadurch gekennzeichnet, daß
der Fundus vor der Behandlung mit dem wäßrigen, sauren Medium zerkleinert wird zu Teilchen mit einer Teilchengröße kleiner 1 cm.

9. Lipase,
mit einem Molekulargewicht von 49 000 Dalton, gemäß dem Verfahren von Laemmli, wovon 9000 Molmassen auf Zucker und 40 000 Molmassen auf ein Protein entfallen,
das nachstehende Anzahl der Aminosäure-Typen aufweist:
Asp. Asn: 45; Thr: 19; Ser: 28; Glx: 30; Pro: 29; Gly: 25; Ala: 28; Val: 27; Met: 8; Ile: 18; Leu: 26; Tyr: 16; Phe: 18; Lys: 17; His: 7; Arg: 10; Cys: 9 und Trp: 6;
das nachstehende N-terminale Sequenz aufweist:
Lys - Ser - Ala - Pro - Thr - Asn - Pro - Glu - Val - Asn -Met - X - Ile - Ser - Glu - Met - Ile - Ser - Tyr - Trp - Gly - Tyr - Pro - Ser - Glu - Lys - Tyr - Glu - Val -Val-, wobei X eine nicht bestimmte Aminosäure bezeichnet;
das eine spezifische Lipaseaktivität nach dem Verfahren von Gargouri über 1000 U/mg Protein aufweist;
das eine maximale Aktivität bei einem pH-Wert von etwa 4,5 aufweist;
dessen Aktivität bei pH-Werten von 3 bis 7 mindestens gleich der Hälfte der maximalen Aktivität ist;
dessen Aktivität nach einer 2 Stunden langen Inkubation bei 37° C und pH 2 erhalten bleibt;
bei dem die an der lipolytischen Aktivität beteiligte Aminosäure Cystein ist;
das gegen Pepsin resistent ist und das von Chymotrypsin sowie Trypsin abgebaut wird;
dessen isoelektrischer pH-Wert zwischen 5,7 und 7,1 liegt; und
das, ausgehend vom Magenfundus eines Kaninchens, leicht durch ein Verfahren nach einem der Ansprüche 1 bis 8 erhältlich ist.

10. Verwendung der Lipase und/oder eines Lipaseextraktes, die nach Anspruch 1 hergestellt oder nach Anspruch 3 definiert worden sind, um ein Medikament gegen mangelhafte Absorptionen von Fetten bei Mensch und Tier zu erhalten.
